# EUROPEAN PATENT APPLICATION

(11) **EP 2 848 696 A1**
(43) Date of publication of application: **18.03.2015**
(21) Application number: 13184458.1
(22) Date of filing: 13.09.2013
(51) Int. Cl.: C12Q 1/37, C07D 487/00, G01N 33/542

(54) **Caspase-1 imaging probes**

(71) Applicant: Sanofi-Aventis Deutschland GmbH, 65929 Frankfurt am Main (DE); Europäisches Laboratorium für Molekularbiologie (EMBL), 69117 Heidelberg (DE); Jozef Stefan Institute, 1000 Ljubljana (SI)
(72) Inventor: Datta, Gopal, 60594 Frankfurt am Main (DE); Schultz, Carsten, 69117 Heidelberg (DE); Plettenburg, Oliver, 65926 Frankfurt am Main (DE); Kurz, Michael, 65926 Frankfurt am Main (DE); Podeschwa, Michael, 65926 Frankfurt am Main (DE); Billen, Guenter, 65926 Frankfurt am Main (DE); Kogler, Herbert, 65926 Frankfurt am Main (DE); Turk, Boris, 1000 Ljubljana (SI); Vizovisek, Matej, 1000 Ljubljana (SI)
(74) Representative: Weiss, Wolfgang

(57) **Abstract**

The present invention relates to molecular probes of the formula (I) 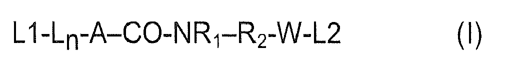 as defined herein that allow for the observation of the catalytic activity capase-1 in *in vitro* assays, in cells or in multicellular organisms, and the use thereof in medicine.

## Description

The present invention relates to molecular probes (substrates) that allow the observation of the activity of caspase-1 in *in vitro* assays, in cells or in multicellular organisms. The invention furthermore relates to the use of such probes (substrates) in medicine, in diagnosis, particularly in imaging living organisms and in the identification of new biomarkers.

### Background of the Invention

Proteolytic enzymes (proteases) cleave or degrade other enzymes or peptides in- and outside of the living cell. Proteases are involved in a multitude of vital processes, many of which are critical in cellular signalling and tissue homeostasis. Aberrant or enhanced activity of proteases is associated with a variety of diseases including cancer, osteoarthritis, atherosclerosis, inflammation and many others.

A particular class of proteases are cysteine proteases. They are characterized by a cysteine residue in the active site which serves as a nucleophile during catalysis. The catalytic cysteine is commonly hydrogen bonded with appropriate neighbouring residues, so that a thiolate ion can be formed. When a substrate is recognized by the protease, the scissile peptide bond is placed in proximity to the catalytic cysteine, which attacks the carbonyl carbon forming a tetrahedral oxoanion intermediate. The amide bond is then cleaved liberating the C-terminal peptide as an amine. The N-terminal portion of the scissile peptide remains in the covalent acyl-enzyme intermediate, which is subsequently cleaved by water, resulting in regeneration of the enzyme. The N-terminal cleavage product of the substrate is liberated as a carboxylic acid.

Caspases are a family of cysteinyl aspartate-specific proteases. The human genome encodes 11 caspases. Eight of them (caspase-2,3,6,7,8,9,10 and 14) function in apoptosis or programmed cell death. They process through a highly regulated signalling cascade. In a hierarchical order, some initiator caspases (caspase-2,8,9 and 10) cleave and activate effector caspases (caspase-3,6 and 7). These caspases are involved in cancers, autoimmune diseases, degenerative disorders and strokes. Three other Caspases (caspase-1, 4 and 5) serve a distinct function: inflammation mediated by activation of a subset of inflammatory cytokines.

Caspase-1 or interleukin-1β-converting enzyme (ICE) is primarily found in monocytic cells. This protease is responsible for the production of the pro-inflammatory cytokines interleukin-1-beta and interleukine-18. Inhibition of caspase-1 has been shown to be beneficial in models of human inflammation disease, including rheumatoid arthritis, osteoarthritis, inflammatory bowel disease and asthma.

For proteolytic enzymes, it is their activity, rather than mere expression level, that dictates their functional role in cell physiology and pathology. Accordingly, molecules that inhibit the activity of caspases are useful as therapeutic agents in the treatment of diseases and diagnostic, e.g., in the development of specific imaging biomarkers that visualize the proteolytic activity as well as their inhibition through drug candidates may accelerate target validation, drug development and even clinical trials (H. Pien et al. Drug Discovery Today, 2005, 10, 259-266). Using activity based imaging reagents, a specific protein or protein family can be readily monitored in complex protein mixtures, intact cells, and even *in vivo.* Furthermore, enzyme class specific probes can be used to develop screens for small molecule inhibitors that can be used for functional studies (D.A. Jeffery, M. Bogyo Curr. Opp. Biotech. 2003, 14, 87-95).

So far, activity based imaging probes incorporating a peptide substrate have been developed to monitor and label in cell based assays caspase-1 (W.Nishii et al., FEBS Letters 2002, 518, 149-153) or caspase-3 (S. Mizukami et al., FEBS Letters 1999, 453, 356-360). Furthermore a near-infrared fluorescent probe has been reported to detect caspase-1 activity in living animals (S. Messerli et al., Neoplasia 2004, 6, 95-105).

To be effective as biological tools, protease inhibitors must be not only very potent but also highly selective in binding to a particular protease. The development of small molecule inhibitors for specific proteases has often started from peptide substrates. Although peptides display a diverse range of biological properties, their use as drugs can be compromised by their instability and their low oral bioavailability and rapid clearance. To be effective drugs, protease inhibitors with reduced peptide-like character, high stability against non selective proteolytic degradation, high selectivity for a given protease, and good bioavailability to the *locus* of protease action are desirable. These requirements led to the idea that a "redesign" of optimized covalent inhibitors (with a chemical scaffold A and an electrophilic warhead B) into a cleavable substrate should be possible. Since the chemical scaffold A can be considered as the determinant of inhibitor selectivity, this approach allows for the transfer of the selectivity or parts of the selectivity of an optimized inhibitor into an activity based chemical probe.

### Detailed Description of the Invention

A new route towards selective activity based probes for cysteine proteases and its application to proteases from the caspases subfamily is disclosed in formula (I) of WO 2009/019115, which document is incorporated herein by reference. Extensive screening of these probes unexpectedly revealed a group of caspase-1 specific probes with advantageous properties.

Thus, the present invention provides molecular probes for cysteine proteases of the formula (I)

L1-Lₙ-A-CO-NR₁-R₂-W-L2 (I)

wherein
A is a group recognizable by caspase-1;
R₁ is hydrogen, hydroxy, C₁-C₁₈-alkyl, preferably C₁-C₆-alkyl, or C₁₄-, C₁₅-, C₁₆-, C₁₇-, C₁₈-alkyl, more preferably C₁-, C₂-, C₃-alkyl, wherein optionally one or more carbon atoms are substituted by halogen; C₃-C₁₈-alkyl, preferably C₃-, C₄-, C₅-, C₁₄-, C₁₅-, C₁₆-, C₁₇-, C₁₈-alkyl wherein one or more carbon atoms are replaced by an oxygen atom; alkylaryl such as C₁-, C₂-, C₃-aryl or -NHCOR₄;
R₂ is a bond or NR₃ wherein R₃ is hydrogen, hydroxy, C₁-C₁₈-alkyl, preferably C₁-C₆-alkyl, more preferably C₁, C₂-, C₃-alkyl, wherein optionally one or more carbon atoms are substituted by halogen; C₃-C₁₈-alkyl, wherein one or more carbon atoms are replaced by an oxygen atom; alkylaryl, such as C₁-, C₂-, C₃-aryl; or -NHCOR₄ or wherein R₁ and R₃ form together with the nitrogen atoms to which they are attached to a 3-, 4-, 5-, 6-membered saturated or unsaturated ring, optionally with one or more, preferably two, additional hetereoatoms selected from oxygen or nitrogen;
R₄, is alkyl, aryl, or alkylaryl, all as defined herein;
W is a straight or branched alkylene group with 1 to 300 carbon atoms, wherein optionally
   a) one or more carbon atoms are replaced by a nitrogen atom carrying a hydrogen atom, and the adjacent carbon atoms are substituted by oxo, representing an amide function -NH-CO-; and/or
   b) one or more carbon atoms are substituted by oxo, representing a carbonyl function -CO;
   c) the bond between two adjacent carbon atoms is a double or triple bond; and/or
   d) one or more carbon atoms are replaced by an ester function -O-CO-; L is a group selected from 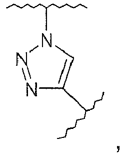 -(NRx)-, -O-, -C=N-, -C(=O)-, -C(=O)-NH-, -NH-C(=O)-, -CRx=CRy-, -C≡C- and phenyl, wherein Rx and Ry are independently H or (C₁-C₆)alkyl, preferably L is -C(=O)-;
L1 and L2 are, independent of each other at least one label; and n is 0 or 1.

Molecular probes according the above formula (I) exhibit unexpected favourable properties:
1. Introduction of the highly unusual hydrazine linker motif unexpectedly resulted in facile cleavage by caspase, and FRET change was concentration dependent. Without being bound to any theory, it is believed that the flanked hydrazine motif allowed the enzyme to get an access of the cleavable bond and thereby improve its cleavage efficiency and kinetic property.
2. The probes of the present invention do not need to make use of a N-terminal linker in order to increase the solubility.
3. Cell-permeability (of particular importance, exemplified by experimental data included herewith) which allows for monitoring caspase-1 in a complex biological micro-environment.
4. Excellent specificity towards caspase-1 over others (other caspases, & cysteine proteases).
5. The probes of the present invention are highly selective, whereby a risk of false positives can be avoided.
6. Incorporation of lipid chain either close to recognition site or far from recognition site induce cellular entry (either by endocytosis or receptor mediated entrance without wishing to be bound to this theory).
7. FIRST-IN-CLASS' probe to serve as biomarker in inflammation based diseases where Caspase-1 is either up regulated or down regulated.
8. From the data (cellular characterization, concentration dependent FRET change, favourable K_{cat}/Kₘ, no-sign of cellular toxicity, and cell-penetration in four different cell lines), it is evident that the inventive probes will be the first "first-in-class" FRET reporter to measure intracellular caspase-1 in complex *in vivo* disease models.

In one embodiment, the molecular probe is of the formula (I) as described herein wherein R₁ is hydrogen and R₂ is a bond, i.e.:

L1-Lₙ-A-CO-NR₁-W-L2 (II)

wherein L1, L, A, R₁, W, n and L2 are defined as described herein.

In a preferred embodiment of formula (I) or (II) as described herein W is a straight or branched alkylene group with 10, 11, 12, 13, 14, 15, 16, 17, 18, 19 or 20 carbon atoms, wherein one or more carbon atoms are replaced by a nitrogen atom carrying a hydrogen atom, and the adjacent carbon atoms are substituted by oxo, representing an amide function -NH-CO-.
In a more preferred embodiment of formula (I) or (II) as described herein W is a straight or branched alkylene group with 10, 11, 12, 13, 14, 15, 16, 17, 18, 19 or 20 carbon atoms, wherein one or two carbon atoms are replaced by a nitrogen atom carrying a hydrogen atom, and the adjacent carbon atoms are substituted by oxo, representing an amide function -NH-CO-, wherein one of said amide functions binds to L2.
In a particular preferred embodiment W is a straight alkylene group with 13 carbon atoms, particularly wherein one or more carbon atoms are replaced as follows -(CH₂)₈-NH-CO-CH₂-NH-CO-.

In another embodiment, the molecular probe is of the formula (I) as described herein wherein R₂ is NR₃, i.e.:

L1-Lₙ-A-CO-NR₁-NR₃-W-L2 (III)

wherein
R₁ and R₃ form together with the nitrogen atoms to which they are attached to a 3-, 4-, 5-, 6-membered saturated or unsaturated ring, optionally with one or more, preferably two, additional hetereoatoms selected from oxygen or nitrogen, or
R₁ and R₃ are independent from each other hydrogen, hydroxy, C₁-C₁₈-alkyl, preferably C₁-C₆-alkyl, more preferably C₁-, C₂-, C₃-alkyl, wherein optionally one or more carbon atoms are substituted by halogen; C₃-C₁₈-alkyl, wherein one or more carbon atoms are replaced by an oxygen atom; alkylaryl such as C₁-, C₂-, C₃-aryl; or -NHCOR₄;
R₄ is alkyl, aryl, alkylaryl, all described herein; and
L1, L, A, W, n and L2 are defined as described herein.

In a preferred embodiment of formula (I) or (III) R₁ and R₃ are each hydrogen.

In another preferred embodiment of formula (I) or (III), R₁ is hydrogen and R₃ is selected from alkyl, alkylaryl, and -NHCOR₄, preferably from alkyl and alkylaryl.

In another preferred embodiment of formula (I) or (III), R₁ is selected from alkyl, alkylaryl, and -NHCOR₄, preferably from alkyl and alkylaryl; and R₃ is hydrogen.

In another preferred embodiment of formula (I) or (III), R₁ and R₃ are each alkyl, alkylaryl, or NHCOR₄, preferably alkyl or alkylaryl.

In another preferred embodiment of formula (I) or (III) R₁ and R₃ form together with nitrogen atoms to which they are attached to a ring as follows: -CH2-NH-NH- or -NH-CH2-NH- or -NH-NH-CH2-.

In another preferred embodiment of formula (I) or (III) R₁ and R₃ form together with nitrogen atoms to which they are attached to a ring as follows: -CH2-CH2-NH- or -NH-CH2-CH2- or -CH2-NH-CH2-.

In another preferred embodiment of formula (I) or (III) R₁ and R₃ form together with nitrogen atoms to which they are attached to a ring as follows: -CH2-CH2-CH2- or -CH2-CH2-CH2-CH2-.

In another preferred embodiment of formula (I) or (III) R₁ and R₃ form together with nitrogen atoms to which they are attached to a ring as follows: -CH2-O-CH2- or -CH2-O-CH2-CH2- or -CH2-CH2-O-CH2-.

In a further preferred embodiment of formula (I) and (III) W is a straight or branched alkylene group with 1, 2, 3, 4, 5, 6, 7, 8, 9, 10, 11, 12, 13 , 13, 14, 15 carbon atoms, wherein one or more carbon atoms are substituted by an oxo function, representing a carbonyl function -C(O), particularly wherein W is a straight alkylene group with 1 carbon atom wherein said carbon atom is substituted by an oxo function, representing a carbonyl function -C(O). Alternatively, W is a straight or branched alkylene group with 20 to 35 carbon atoms, wherein one or more carbon atoms are replaced by a nitrogen atom carrying a hydrogen atom, and the adjacent carbon atoms are substituted by oxo, representing an amide function -NH-CO- and one or more carbon atoms are substituted by oxo, representing a carbonyl function -C(O), particularly wherein W is -C(O)CH(Z)NH-C(O)- wherein Z is (CH₂)_{q}-NHC(O)(CH₂)ₘCH₃, wherein q is 1, 2, 3, 4, 5, 6, 7 and m is 10, 11, 12, 13, 14, 15, 16, 17, 18, 19, 20, preferably wherein q=4 and m=14. Alternatively W is a straight or branched alkylene group with 5-10 carbon atoms, wherein one or more carbon atoms are substituted by oxo, representing a carbonyl function -C(O) and one or more carbon atoms are replaced by an ester function -O-CO- particularly wherein W is -C(O)(CH₂)ₚC(O)OCH₂- and p is 1, 2, 3, 4, 5, 6, preferably 3.

In a preferred embodiment, L is -C(=O)-.

The label L1 and L2 of the substrate can be chosen by those skilled in the art dependent on the application for which the probe is intended.

The label L1 and L2 is independently of each other a spectroscopic probe such as a fluorophore; a quencher or a chromophore; a magnetic probe; a contrast reagent; a molecule which is one part of a specific binding pair which is capable of specifically binding to a partner; or a molecule possessing a combination of any of the properties listed above; or a positively charged linear or branched polymer, preferably L1 and L2 is independently of each other a fluorophore.

In a preferred embodiment, L1 and L2 are independently from each other a coumarin derivative, particularly a substituted coumarin such as 7-alkoxycoumarin, e.g 7-methoxycoumarin, 7-hydroxycoumarin, 6-halogen-,7-hydroxycoumarin, or N, N-dialkylcoumarin.

In particular, L1 may be selected from 7-methoxycoumarin, 6-bromo-,7-hydroxycoumarin, and 7-hydroxycoumarin.

In particular, L2 is selected from 7-N,N-dialkylcoumarin, preferably 7-N,N-diethylcoumarin or 7-N,N-dioctanylcoumarin.

These can be advantageously combined as follows:

| L1 | L2 |
|---|---|
| 7-methoxycoumarin | 7-N,N-diethylcoumarin |
| 7-methoxycoumarin | 7-N,N-dioctanylcoumarin |
| 6-bromo-,7-hydroxycoumarin | 7-N,N-diethylcoumarin |
| 7-hydroxycoumarin | 7-N,N-diethylcoumarin |

A probe which consists of intramolecularly quenched fluorescent probes comprising a polymeric backbone and a plurality of fluorochromes covalently linked *via* scaffold A to the backbone at a density which leads to fluorescent quenching is preferred.

Another preferred embodiment of the present invention is the use of a dendritic macromolecule onto which two or more fluorophores are covalently linked *via* scaffold A at a density which leads to fluorescent quenching. The use of a polymeric probe has the advantage of localized probe delivery (targeting) and a prolonged circulation time in the blood stream of an animal or humans. Polymer conjugation alters the biodistribution of low-molecular-weight substances, enabling tumour-specific targeting (by the enhanced permeability and retention effect (EPR effect)) with reduced access to sites of toxicity and the combination of polymer conjugates with low-molecular-weight imaging probes is a most preferred embodiment of the present invention for imaging of multicellular organisms including mammals such as mice, rats etc.. The polymeric backbone can consist of any biocompatible polymer and may comprise a polypeptide, a polysaccharide, a nucleic acid or a synthetic polymer. A comprehensive summary of polymers useful in the context of the present invention can be found in M.J. Vincent et al. Trends Biotech. 2006, 24, 39-47 and R. Duncan, Nature Reviews Cancer, 2006, 688-701. A further description of polymers useful in the context of the present invention is disclosed in WO99/58161. The polymeric or dendrimeric probe can comprise protective chains covalently linked to the backbone or the dendritic molecule. Protective chains include polyethylene glycol, methoxypolyethyleneglycol and further copolymers of ethyleneglycol.

The probe of the present invention can additionally comprise a targeting moiety such as an antibody, an antibody fragment, a receptor-binding ligand, a peptide fragment or a synthetic protein inhibitor.

Label L1 and L2 can further be positively charged linear or branched polymers. Said polymers are known to those skilled in the art to facilitate the transfer of attached molecules over the plasma membrane of living cells. This is especially preferred for substances which otherwise have a low cell membrane permeability or are in effect impermeable for the cell membrane of living cells. A non cell permeable chemical probe will become cell membrane permeable upon conjugation to such a group L1 or L2. Such cell membrane transport enhancer groups L1 and L2 comprise, for example, a linear poly(arginine) of D- and/or L-arginine with 6 - 15 arginine residues, linear polymers of 6 - 15 subunits each of which carry a guanidinium group, an oligomer or a short-length polymer of from 6 to up to 50 subunits, a portion of which have attached guanidinium groups, and/or parts of the sequence of the HIV-tat protein, for example the subunit Tat49-Tat57 (RKKRRQRRR in the one letter amino acid code). A linear poly(arginine) of D- and/or L-arginine with 6 - 15 arginine residues is preferably utilized as polymeric label, particularly in case L1 is one member and L2 is the other member of two interacting spectroscopic probes L1 / L2, such as in a FRET pair.

Most preferred as label L1 and/or L2 are spectroscopic probes. Most preferred as label L2 are molecules representing one part of a spectroscopic interaction pair with L1, furthermore a label which is capable of specifically binding to a partner and molecules covalently attached to a solid support.

Particularly preferred are labels such that L1 is one member and L2 is the other member of two interacting spectroscopic probes L1 / L2, wherein energy can be transferred non-radiatively between the donor and acceptor (quencher) through either dynamic or static quenching. Such said pair of label L1 / L2 changes its spectroscopic properties upon reaction/cleavage through the corresponding caspase protease. An example of such a pair of label L1 / L2 is a FRET (Förster resonance energy transfer) pair, e.g. a pro-fluorescent probe covalently labelled at one end (e.g. L1) with a donor (reporter), and the another position (L2) with an acceptor (quencher), or *vice versa*.

In particular, L1 is a donor (reporter) and L2 is an acceptor (quencher), or L1 is a quencher and L2 is a reporter. In using this probe, the reaction of caspase-1 with the probe will lead to a change in fluorescence. The reporter-quencher distance within the double labelled substrate is changed upon reaction with the protease leading to a spatial separation of reporter and quencher which causes the appearance of fluorescence or change of the emission wavelength. A broad selection of reporter groups may be used as label L1 or L2, respectively, including e.g. near infra-red emitting fluorophores. The substrate containing reporter and quencher remains dark until it reacts with the protease, whereupon the reaction mixture is "lit up" switching on the fluorophore emission, since the reporter label and the quencher label are now spatially separated. Fluorescence quenching and energy transfer can be measured by the emission of only one of the two labels, the quenched or energy donor label. When energy transfer occurs and the energy accepting label is also fluorescent, the acceptor label fluorescence can also be measured. A donor label of these two interacting label can be chosen from chemoluminescent donor probes which eliminates the need of an excitation lamp and reduces acceptor background fluorescence. The mentioned particular method using such double-labelled substrates is useful to determine reaction kinetics based on fluorescence time measurements, and may be applied *in vivo* as well as *in vitro.*

Alternatively, the label L2 may be attached or attachable to a polymer. Linear poly(arginine) of D- and/or L-arginine with 6 - 15 arginine residues is preferably utilized as polymeric label for a L1 / L2 FRET pair.

Particular preferred combinations are two different affinity label, especially a pair of spectroscopic interacting label L1 / L2, e.g. a FRET pair. An affinity label is defined as a molecule which is one part of a specific binding pair which is capable of specifically binding to a partner. A specific binding pair considered is e.g. biotin and avidin or streptavidin furthermore methotrexate, which is a tight-binding inhibitor of the enzyme dihydrofolate reductase (DHFR).

Appropriate pairs of reporters and quenchers can bee chosen by those skilled in the art. Typically reporter and quencher are fluorescent dyes with large spectral overlap as, for example, fluorescein as a reporter and rhodamine as a quencher. Other quenchers are gold clusters, and metal cryptates.

A second class of quenchers used in this invention are "dark quenchers", i.e. dyes without native fluorescence having absorption spectra that overlap with the emission spectra of common reporter dyes leading to maximal FRET quenching. Furthermore pairs of dyes can be chosen such that their absorption bands overlap in order to promote a resonance dipole-dipole interaction mechanism within a ground state complex (static quenching).

Particular fluorophores and quenchers considered are: Alexa dyes, including Alexa 350, Alexa 488, Alexa 532, Alexa 546, Alexa 555, Alexa 635 and Alexa 647 (US5696157, US6130101, US6716979); dimethylaminocoumarin (e.g. 7-dimethylaminocoumarin-4-acetic acid succinimidyl ester supplied as product D374 by Invitrogen, CA 92008, USA); quenchers QSY 35, QSY 9 and QSY 21 (Invitrogen, CA 92008, USA); Cyanine-3 (Cy 3), Cyanine 5 (Cy 5) and Cyanine 5.5 (Cy 5.5) (Amersham - GE Healthcare, Solingen, Germany); BHQ-1, BHQ-2 and BHQ-3 (Black Hole QuencherTM of Biosearch Technologies, Inc., Novato, CA 94949, USA); fluorophores ATTO 488, ATTO 532, ATTO 600 and ATTO 655 and quenchers ATTO 540Q and ATTO 612Q (Atto-Tec, D57076 Siegen, Germany); fluorophores DY-505, DY-547, DY-632 and DY-647 (Dyomics, Jena, Germany); 5/6-carboxyfluorescein, tetramethylrhodamine, 4-dimethylaminoazobenzene-4'-sulfonyl derivatives (Dabsyl) and 4-dimethylaminoazobenzene-4'-carbonyl derivatives (Dabcyl).

Preferably, one label L1 / L2 is a fluorophor selected from Alexa 350, dimethylaminocoumarin, 5/6-carboxyfluorescein, Alexa 488, ATTO 488, DY-505, 5/6-carboxyfluorescein, Alexa 488, Alexa 532, Alexa 546, Alexa 555, ATTO 488, ATTO 532, tetramethylrhodamine, Cy 3, DY-505, DY-547, Alexa 635, Alexa 647, ATTO 600, ATTO 655, DY-632, Cy 5, DY-647 Cy 5.5, and the other label L1 / L2 is a quencher selected from Dabsyl, Dabcyl, BHQ 1, QSY 35, BHQ 2, QSY 9, ATTO 540Q, BHQ 3, ATTO 612Q, QSY 21.

These can be advantageously combined in the following combinations:

| Fluorophore | Quencher |
|---|---|
| • Alexa 350, dimethylaminocoumarin, 5/6-carboxyfluorescein, Alexa 488, ATTO 488, DY-505 | • Dabsyl, Dabcyl, BHQ 1, QSY 35 |
| • 5/6-carboxyfluorescein, Alexa 488, Alexa 532, Alexa 546, Alexa 555, ATTO 488, ATTO 532, tetramethylrhodamine, Cy 3, DY-505, DY-547, | • BHQ 2, QSY 9, ATTO 540Q |
| • Alexa 635, Alexa 647, ATTO 600, ATTO 655, DY-632, Cy 5, DY-647 Cy 5.5 | • BHQ 3, ATTO 612Q, QSY 21 |

In one embodiment, the group A recognizable by caspase-1 is characterized by its specificity towards caspase-1.

In a further embodiment, the molecular probe as described herein is selective for caspase-1.

Group A is preferably the main determinant for specificity towards caspase-1, as shown in Table 1. Activity-based probes of the present invention show selectivity for caspase-1 of the factor 1000 to 1, preferably a factor 10 to 1, wherein selectivity is defined by the relative turnover number (turnover number with enzyme 1 versus turnover number with enzyme 2) at a preferred substrate concentration. The relative turnover number is determined for each enzyme pair by dividing the turnover number of the enzyme of interest (enzyme 1) by the turnover number of another enzyme against which selectivity is desired (enzyme 2). For *in vivo* applications high selectivity is desired at low (e.g. micromolar or submicromolar) substrate concentrations.

Preferably, the probe of the formula (I), (II), or (III) comprises a group A being an inhibitor of caspase-1. The preparation of scaffolds A having caspase-1 inhibitory activity is for example described in US5670494; WO9526958; WO9722619; WO9816504; WO0190063; WO03106460; WO03104231; WO03103677; W. G. Harter, Bioorg. Med. Chem. Lett. 2004, 14, 809-812; Shahripour et al., Bioorg. Med. Chem. Lett. 2001, 11, 2779-2782; Shahripour et al., Bioorg. Med. Chem. 2002, 10, 31-40; M. C. Laufersweiler et al., Bioorg. Med. Chem. Lett. 2005, 15, 4322-4326; K. T. Chapman, Bioorg. Med. Chem. Lett. 1992, 2, 613-618; Dolle et al., J. Med. Chem. 1997, 40, 1941-1946; D. L. Soper et al., Bioorg. Med. Chem. Lett. 2006, 16, 4233-4236; D. L. Soper et al., Bioorg. Med. Chem. 2006, 14, 7880-7892; D. J. Lauffer et al., Bioorg. Med. Chem. Lett. 2002, 12,1225-1227; and C. D. Ellis et al., Bioorg. Med. Chem. Lett. 2006, 16, 4728-4732. More preferred, the probe of the formula (I), (II), or (III) comprises a group A wherein A is selected from compound 1-28 of Table 1 of WO 2009/019155. Most preferred, the compound of the formula (I), (II), or (III) is a probe wherein A is selected from the following preferred scaffolds A (Table 1):

**Table 1 - Examples of selective probes (I) for caspase-1**

| | |
|---|---|
| 1. | 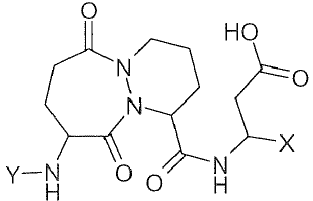 |
| | preferably in the configuration: |
| | 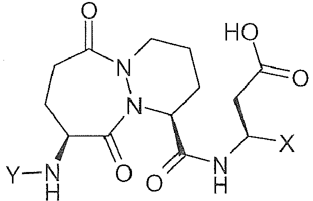 |
| 2. | 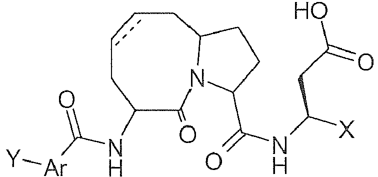 |
| | wherein Ar is an aryl or heteroaryl group selected from phenyl, benzothiophene, isoquinolyl, cinnamyl, naphthyl, which is optionally once or independently twice substituted by methoxy, chloro, methyl, CF₃, and wherein |
| | 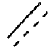 |
| | means either a single or a double bond. |
| 3. | 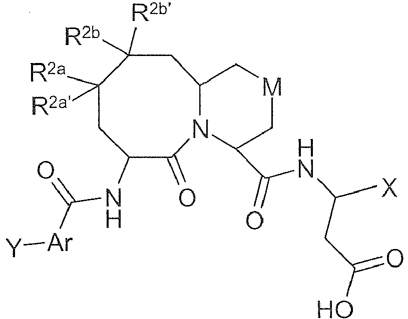 |
| | preferably in the all-(S) configuration, |
| | wherein Ar is aryl or heteroaryl; |
| | M is CH₂, O or NR₉ wherein Rg is hydrogen or (C₁-C₆)alkyl, aryl, heteroaryl, heterocyclyl; |
| | R^{2a}, R^{2a'}, R^{2b} and R^{2b'} are each independently hydrogen, hydroxyl, N(R⁶)₂, halogen, (C₁-C₄)alkyl, (C₁-C₄)alkoxy, and mixtures thereof wherein R⁶ is hydrogen, (C₁-C₆)alkyl, cycloalkyl, (C₆-C₁₀)aryl; or R^{2a} and R^{2b} can taken together to form a double bond. |
| 4. | 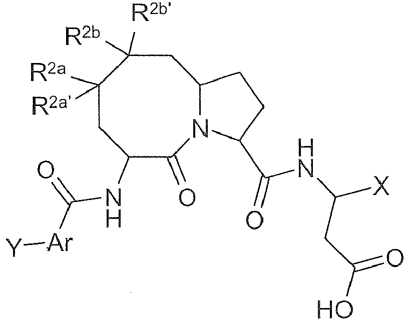 |
| | preferably in the all-(S) configuration, |
| | wherein Ar is aryl or heteroaryl; |
| | R^{2a}, R^{2a'}, R^{2b} and R^{2b'} are each independently hydrogen, hydroxyl, N(R⁶)₂, halogen, (C₁-C₄)alkyl, (C₁-C₄)alkoxy, and mixtures thereof wherein R⁶ is hydrogen, (C₁-C₆)alkyl, cycloalkyl, (C₆-C₁₀)aryl; or R^{2a} and R^{2b} can taken together to form a double bond. |

wherein the variables in compounds 1. to 4. are defined as indicated in the definition next to the respective compound; X is -CO-NR₁-R₂-W-L2; Y is L1-Lₙ-; and R₁, R₂, Lₙ, W, L1 and L2 are defined as described herein.

In a particularly preferred embodiment, A is 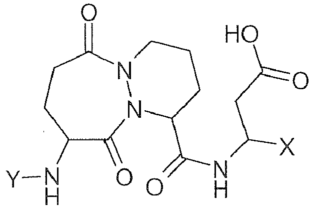 particularly in the configuration of 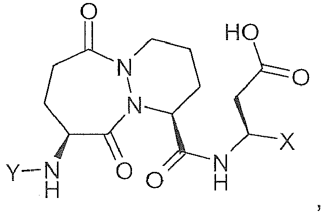 wherein in each of the above compounds X is -CO-NR₁-R₂-W-L2; Y is L1-Lₙ-; and R₁, R₂, Lₙ, W, L1 and L2 are defined as described herein.

The following definitions apply if not otherwise stated:
Alkyl means a straight or branched chain hydrocarbon having 1 to 6 carbon atoms. Examples of (C₁-C₆)alkyl groups are methyl, ethyl, propyl, isopropyl, isobutyl, butyl, tert-butyl, sec-butyl, pentyl, and hexyl. Examples of (C₁-C₃)alkyl groups are methyl, ethyl, propyl, and isopropyl.

Halogen is F, Cl, Br, or I.

Acyl is defined as a group -C(=O)alkyl.

Aryl is defined as an aromatic hydrocarbon having 6 to 10 carbon atoms. Examples of aryl groups include phenyl and naphthyl.

Heteroaryl is defined as an aryl group wherein one or more carbon atom of the aromatic hydrocarbon has been replaced with a heteroatom. The term "heteroatom" as used herein includes oxygen, nitrogen, sulfur, and phosphorus. Examples of heteroaryl groups include furan, thiophene, benzothiophene, pyrrole, thiazole, pyridine, pyrimidine, pyrazine, benzofuran, indole, coumarin, quinoline, isoquinoline, and naphthyridine.

Cycloalkyl means a cyclic alkyl group having 3 to 10 carbon atoms. Examples of cycloalkyl groups include cyclopropane, cyclobutane, cyclopentane, and cyclohexane.

Heterocycle or heterocylyl means a cycloalkyl group on which one or more carbon atom has been replaced with a heteroatom. Examples of heterocycles include piperazine, morpholine, and piperidine.

The aryl, heteroaryl, or cycloalkyl groups may be substituted with one or more substituents, which can be the same or different. Examples of suitable substituents include alkyl, alkoxy, thioalkoxy, hydroxy, halogen, trifluoromethyl, amino, alkylamino, dialkylamino, NO₂, CN, CO₂H, CO₂alkyl, SO₃H, CHO, C(=O)alkyl, CONH₂, CONH-alkyl, CONHR_{q}, C(=O)N(alkyl)₂, (CH₂)ₙNH₂, OH, CF₃, O(C₁-C₆)alkyl, (CH₂)ₙNH-alkyl, NHR_{q}, NHCOR_{q}, phenyl, where n is 1 to 5 and Rq is hydrogen or (C₁-C₆)alkyl.

The activity based probes of the present invention may be synthesized by using appropriate protecting group chemistry known in the art to build up the central scaffold A and to attach label L1 or L2 to this unit optionally *via* a group L and a group -C(O)-NH-. Appropriate building blocks as well as labels such as fluorophores, e.g. substituted coumarins as described herein or FRET-pairs such as the cyanine-dyes (e.g. Cy3 B, Cy 5.5, Cy 7) are commercially available (e.g. Sigma-Aldrich, GE-Healthcare). For a subset of probe, described in this invention, the solid-phase synthesis method is particularly useful (B. J. Merrifield, Methods in Enzymology 1997, 289, 3-13). Depending on the synthetic requirements, attachment linker, quencher or fluorophore may be done on the solid support or by solution phase chemistry.

In general, reactive side chain residues on the central scaffold A and optionally the group L will be protected and liberated sequentially for further modification with the subunits L1 and L2 respectively. Conjugation of these subunits can be accomplished by known methods of chemical synthesis. Particular useful is the reaction between a dye active ester and a primary amine group of the scaffold A to connect both units *via* an amide bond. Intermediates as well as final probe molecules may be purified by high performance liquid chromatography (HPLC) and characterized by mass spectrometry and analytical HPLC before they are used in labelling and imaging experiments.

The present invention is illustrated in the following paragraph by several but non-limiting examples:
In a preferred embodiment, the probe of the formula (I) comprises a scaffold A which is derived from a caspase-1 inhibitor (Table 1, compound 1) bearing a chromophore at the C-terminal side and at the N-terminal. Appropriate chromophores are chosen in a way that their spectral properties are suitable for fluorescence resonance energy transfer (FRET). Chromophores can be fluorescent or non fluorescent. In principle, a broad variety of chromophores may be used in the present invention, as long as the central requirement that is a spectral change after proteolytic cleavage of a peptide bond is met. The attachment of such interacting chromophores and the central scaffold is made optionally *via* linker units L.

Preferably, the fluorophores are chosen from the group of coumarin derivatives as described herein.

More preferred is a probe of the formula (I) or (III) based on a scaffold A (Table 1, compound 1) as follows (Scheme 1):

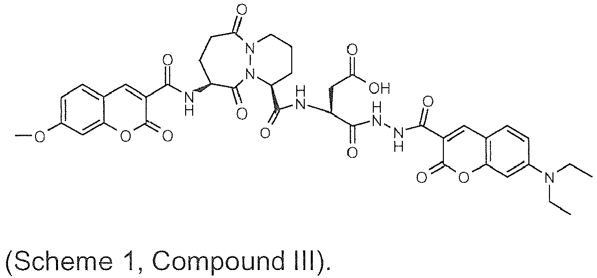

A further preferred probe includes the same formula (I) or (III) based on a scaffold A as follows (Scheme 2):

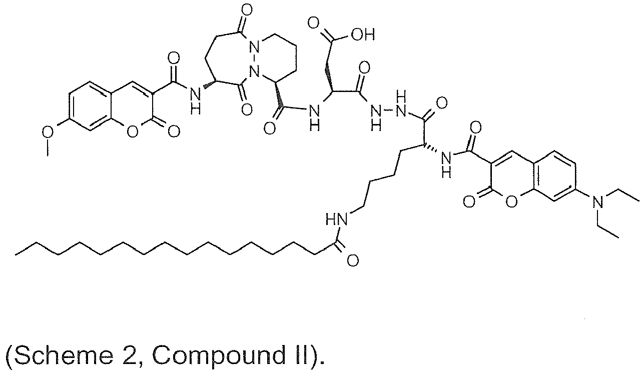

A further preferred probe includes formula (I) or (III) and the same scaffold A as follows (Scheme 3):

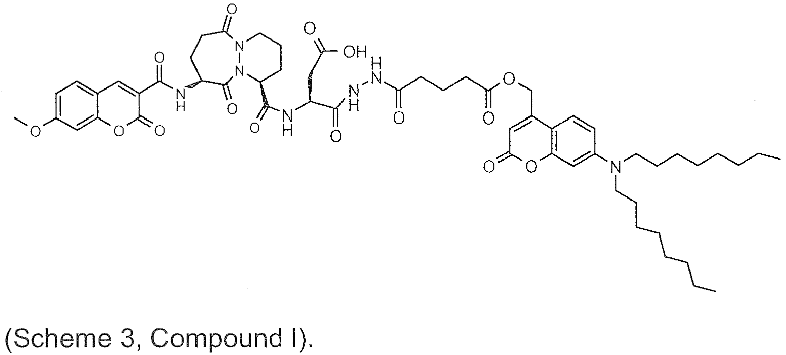

A further preferred probe includes formula (I) or (III) and the same scaffold A is as follows (Scheme 4):

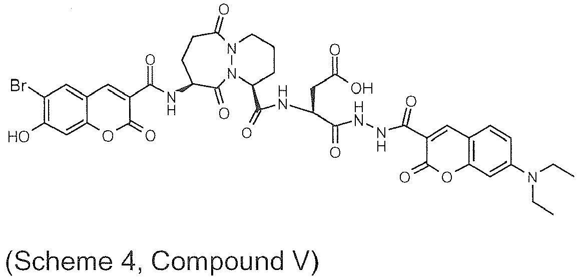

A further preferred embodiment includes formula (I) or (III) and the same scaffold A as follows (Scheme 5):

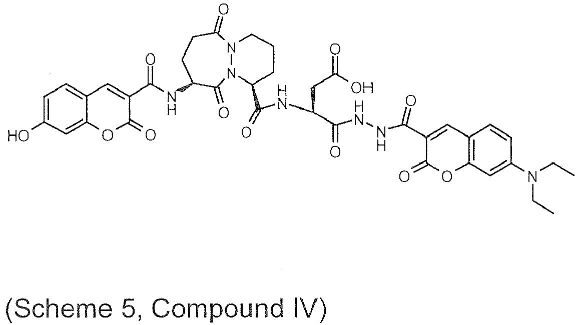

More preferred is also a probe of the formula (I) or (II) based on a scaffold A (Table 1, compound 1) is as follows (Scheme 6):

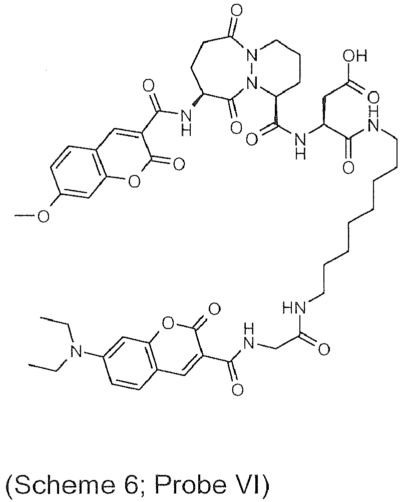

A further preferred probe of the formula (I) or (II) based on a scaffold A (Table 1, compound 1) is as follows (Scheme 7):

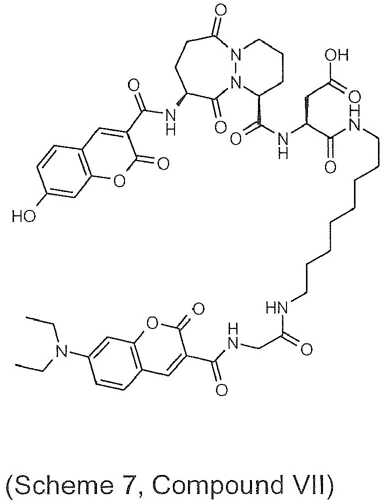

The invention further relates to a method for the design of a molecular probe for the observation of the catalytic activity of caspase-1 in *in vitro* assays, in cells or in multicellular organisms, characterized in transforming an inhibitor for caspase-1 into a selective imaging probe for caspase-1. To achieve this, the electrophilic groups of certain known caspase-1 inhibitors, e.g. Pralnacasan (Siegmund B and Zeitz M (2003), Drugs 6,, 154-158) were replaced with a scissile bond as reflected by formulas (I), (II), or (III). Compounds may be synthesized in a way that a detectable signal is generated by the enzymatic (e.g. proteolytic) activity of a specific target. Preferred compounds may be synthesized in a way that there is a FRET change. Particularly, preferred probes comprise fluorophores (e.g. appropriate internally quenched FRET-pairs) linked to (i) the specificity determinant A at the N-terminal portion of the scissile bond and (ii) at the C-terminal portion of the scissile bond. The invention allows for the transfer elements of desirable and previously optimized properties of known inhibitors into novel activity based probes.

*In vitro,* the reaction of caspase-1 with the probe of the invention can generally be either performed in cell extracts or with purified or enriched forms of caspase-1. For *in vivo* application, the reporters are preferably emitters in the near infra red (NIR) region because that region is absent of interfering biofluorescence. Known cyanine NIR dyes matching these requirements are preferably incorporated in the substrates of the present invention.

The molecular architecture of probes of the formulas (I), (II) or (III) consists of a central scaffold A bearing an amide or hydrazide functional group and two subunits L1L and L2W respectively. L2W is, as shown in formula (I), always connected to scaffold A *via* an amide or hydrazide group since the amide or hydrazide group can be cleaved by the caspase enzyme. Appropriate functional groups for the attachment of subunits L1 to scaffold A can be chosen by those skilled in the art, and examples are given below. The specific functional groups L' in the precursor compound can be placed on the scaffold A or on L1 for the attachment of suitable L1 subunits to yield the group L within the compound of the formulas (I), (II) or (III) are limited only by the requirement of the synthesis strategy and the final use of such substrate as an activity based imaging reagent. Thus their selection will depend upon the specific reagents chosen to build the desired substrates. Examples of functional groups L' which can be provided on scaffold A or on L1 to connect A with the subunit L1R1 include fluoro, chloro, bromo, cyano, nitro, amino, azido, alkylcarbonylamino, carboxy, carbamoyl, alkoxycarbonyl, aryloxycarbonyl, carbaldehyde, hydroxy, alkoxy, aryloxy, alkylcarbonyloxy, arylcarbonyloxy, a carbon-carbon double bond, a carbon-carbon triple bond, and the like. Preferable examples include amino, azido, hydroxy, cyano, carboxy, carbamoyl, carbaldehyde, or a carbon-carbon double or a carbon-carbon triple bond. Most preferred examples is carboxy. Thus, L is preferably a group selected from 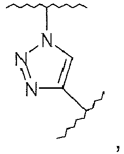 -(NRx)-, -O-, -C=N-, -C(=O)-, -C(=O)-NH-, -NH-C(=O)-, - CRx=CRy-, -C=C- and phenyl, wherein Rx and Ry are independently H or (C₁-C₆)alkyl, most preferably L is -C(=O)-.

In particular, the preferred synthesis of a probe of formulas (I), (II) or (III) is as described in the examples and may make use of orthogonally protected functional groups. Such a choice of protective groups allows for a separate deprotection so that each released functionality in turn can be further chemically manipulated towards the attachment of the corresponding subunits to scaffold A. Appropriate protecting groups for the envisioned functionalities can be chosen by those skilled in the art, and are e.g. summarized in T.W. Greene and P.G.M. Wuts in "Protective Groups in Organic Synthesis", John Wiley & Sons, New York 1991.

The probes of the present invention are used in medicine. In particular, with an increasing complexity of disease biology, understanding the patho-physiological condition is of utmost importance and as well as biggest challenge for drug discovery. In order to increase the chance of success for a compound from bench-to-market, understanding the late stage complication and finding out the disease pathway, mechanism of action is crucial. In addition, early-diagnosis of disease and monitoring of disease treatment are of primary importance. The probes of the current invention may assist the above challenges since the monitor the activity of caspase-1 and may lead to the identification of biomarkers.

Caspase-1 is the major proinflammatory caspase that is critically involved in the activation of prolL-1beta and prolL-18, thereby playing a major role in inflammation. Caspase-1, produced as inactive zymogen, gets activated on a multiprotein scaffold, inflammasome, in cells challenged with specific microbial or danger signals. However, complete understanding of caspase-1 activation and its role in inflammation has remained difficult mainly due to the lack of tools to monitor not only its abundance but also its activity. So for all the disease areas where inflammation is either up-regulated or downregulated, monitoring caspase-1 *in*-*vivo* activity will certainly lead to the identification of biomarkers crucial for inflammatory diseases such as sepsis, endotoxic shock, actute pancreatitis, inflammatory bowel disease, ischaemia of brain or heart, rheumatoid arthritis, osteoarthritis, diabetes, graft vs. host disease, and/or multiple myeloma.

Thus, one aspect of the invention relates to the use of the inventive probes for the identification of biomarkers *in vitro* and *in vivo,* preferably biomarkers for inflammatory diseases.

A further aspect of the invention relates to the inventive probes for use in the prevention, treatment and alleviation of caspase-1 related diseases such as inflammatory diseases. Preferred inflammatory diseases in this context are sepsis, endotoxic shock, acute pancreatitis, inflammatory bowel disease, ischaemia of brain or heart, rheumatoid arthritis, osteoarthritis, diabetes, graft vs. host disease, and/or multiple myeloma.

The probes of the present invention are used in diagnosis, e.g., in the context of molecular imaging in vitro, in cell-culture experiments, ex-vivo experiments or in imaging a living organism (*in vivo*), including screening and whole animal imaging. Mostly preferred are imaging modalities such as optical imaging and magnetic resonance imaging (MRI).

The probes of the present invention are intended to be used for diagnostic imaging of caspase-1 activity. Most preferred are applications which provide methods of monitoring the effect of a drug or drug-like substance towards caspase-1. Administration of such a drug or drug like substance should have a measurable effect to the signal from the probe of the present invention.

A further most preferred aspect of the probes of the present invention is their use as imaging reagents in surgical guidance and to monitor the effect of medical treatment. Surgical guidance includes the detection of tumour margin and detection of progression of tumour metastasis.

Therefore, a further aspect of the present invention is method of imaging a living organism, comprising:
(a) administering to said organism a probe of the formula (I),
(b) exposing said organism to electromagnetic radiation which excites a non-quenched fluorophore to produce a detectable signal, and
(c) detecting said signal and creating an image thereby.

Alternatively, the method of imaging a living organism comprises:
a) administering to said organism a probe of the formula (I),
(b) exposing said organism to electromagnetic radiation which excites a fluorophore to produce a detectable signal; and
(c) detecting said signal and creating an image thereby.

A "living organism" may be any live cell or whole organism comprising caspase-1, preferably the living organism is a mammal, e.g. a mouse or a rat.

### Brief Description of the Figures

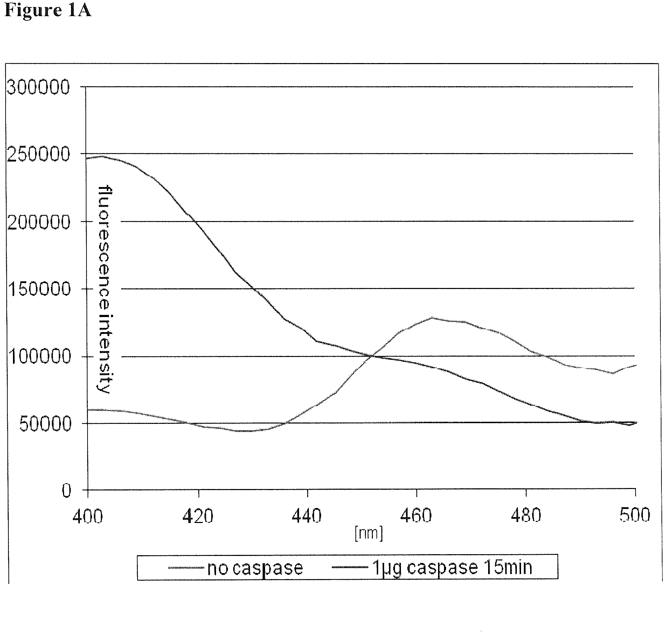

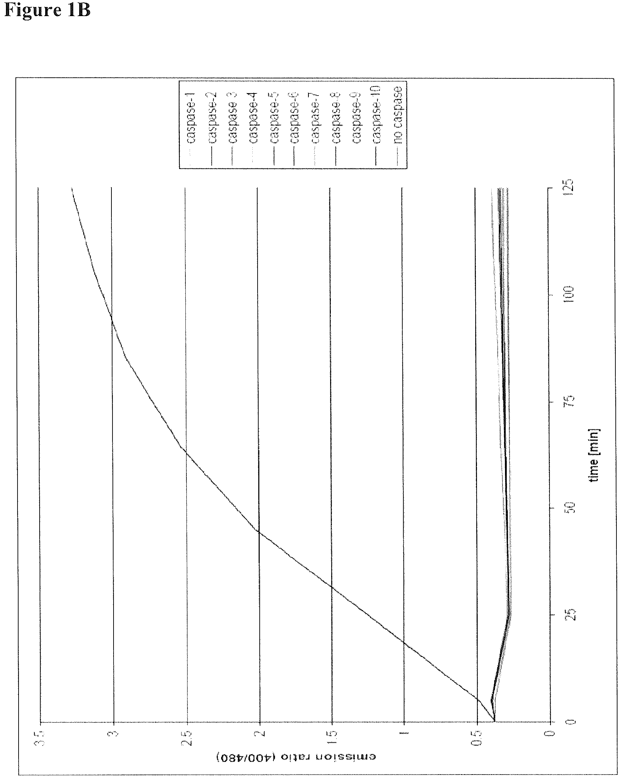

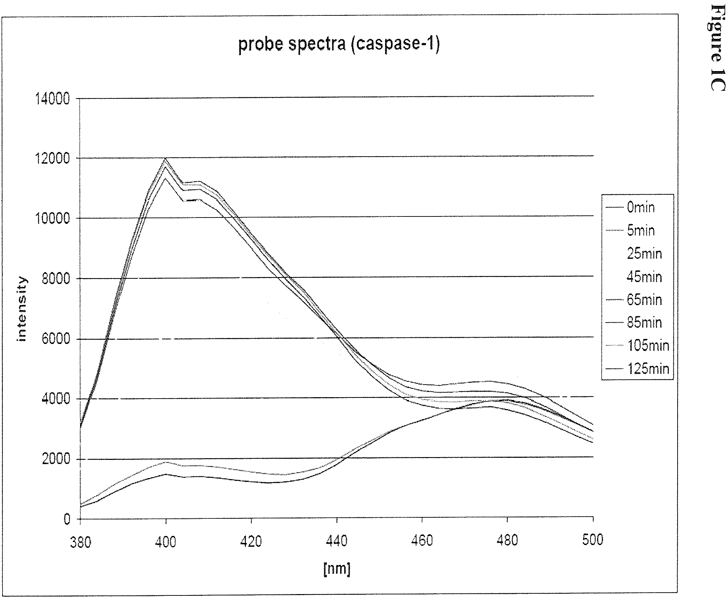

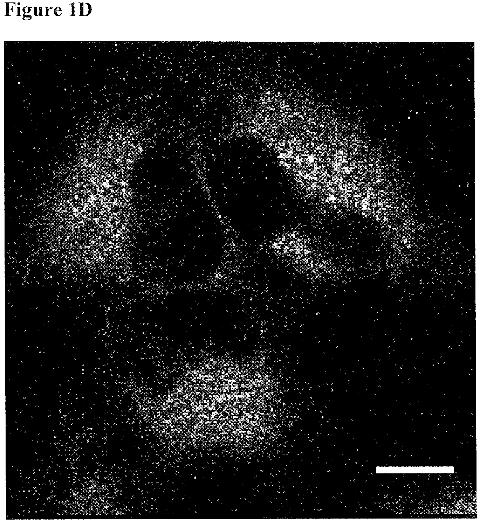

Figure 1: A shows the change of fluorescence intensity of probe III in response to cleavage by caspase-1. The cleavage occurs in an enzyme dose-dependent manner. The FRET change is about 10-fold; B shows the specificity of Probe III towards caspase-1; C shows the probe spectra of cleavage of probe III by caspase-1; D shows the distribution of probe III in Hela cells, cells were incubated with 1 µM of probe III for up to 2h at 37°C.

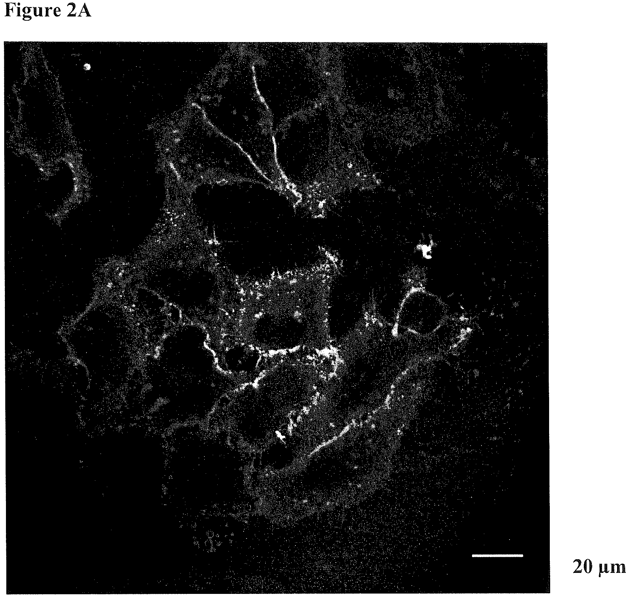

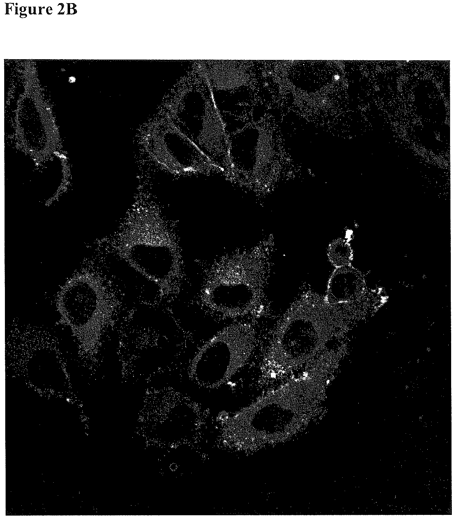

Figure 2: A shows Hela cells 12 min. after incubation of the cells for 5-10 min. at RT with 1 µM probe II; B shows the Hela cells 32 min. after incubation with probe II under the same conditions.

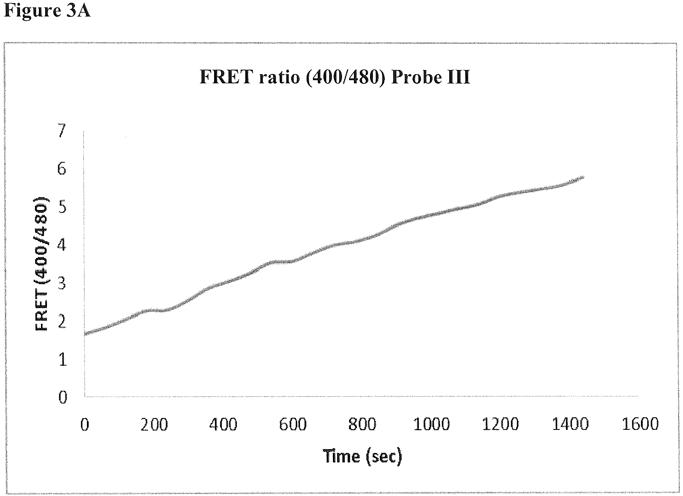

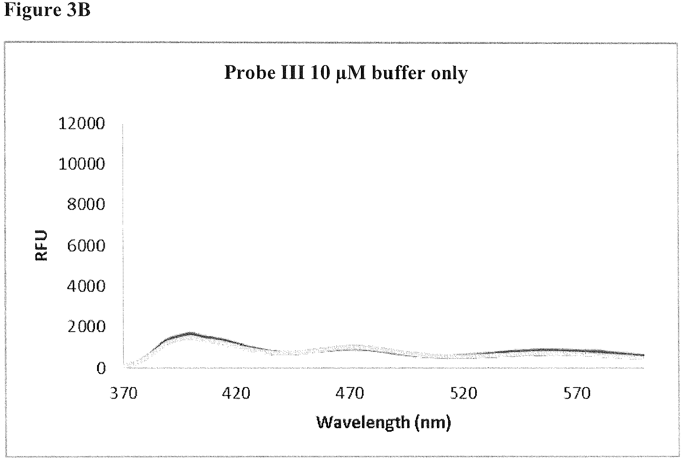

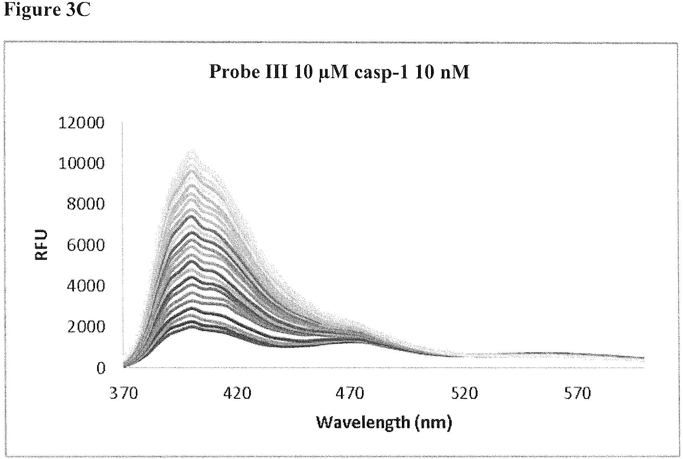

Figures 3A-C: show the in vitro cleavage of 10 µM probe III in the presence of 10 nM caspase-1. The probe is cleaved very efficiently. The E/S ratio was 1/1000, 25 cycles: 1 min. intervals.

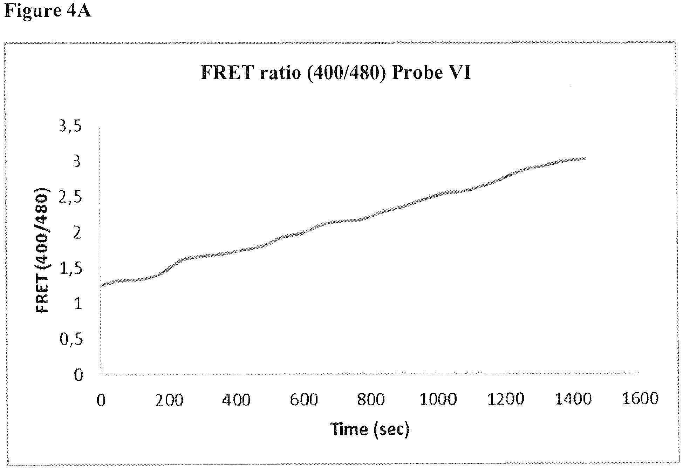

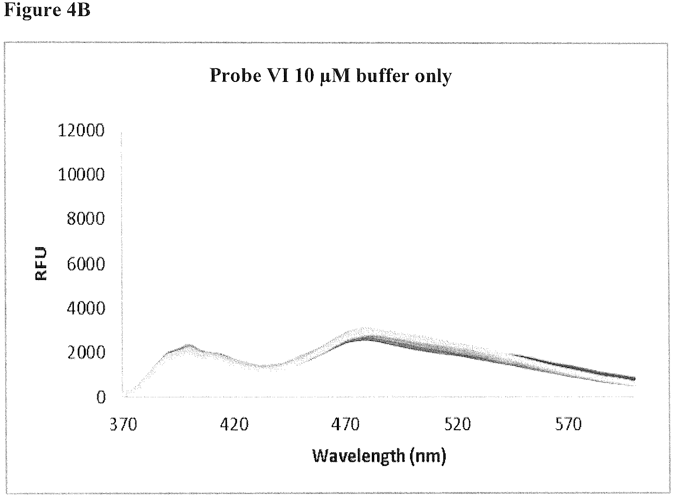

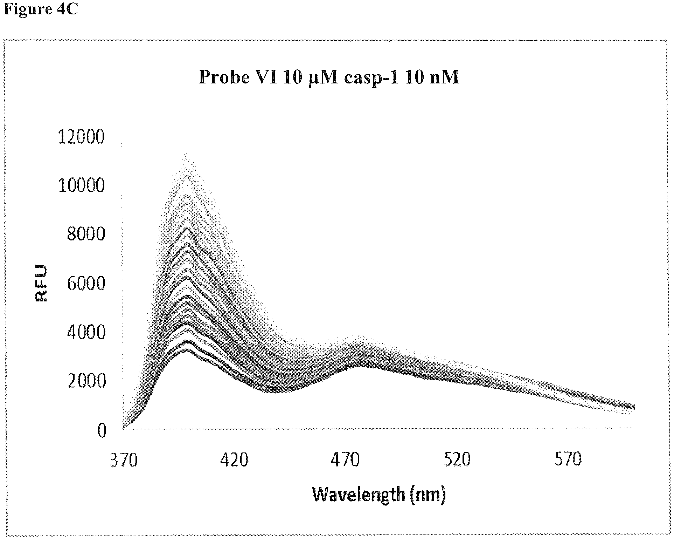

Figures 4A-C: show the in vitro cleavage of 10 µM probe VI in the presence of 10 nM caspase-1. The probe is cleaved very efficiently. The E/S ratio was 1/1000, 25 cycles: 1 min. intervals.

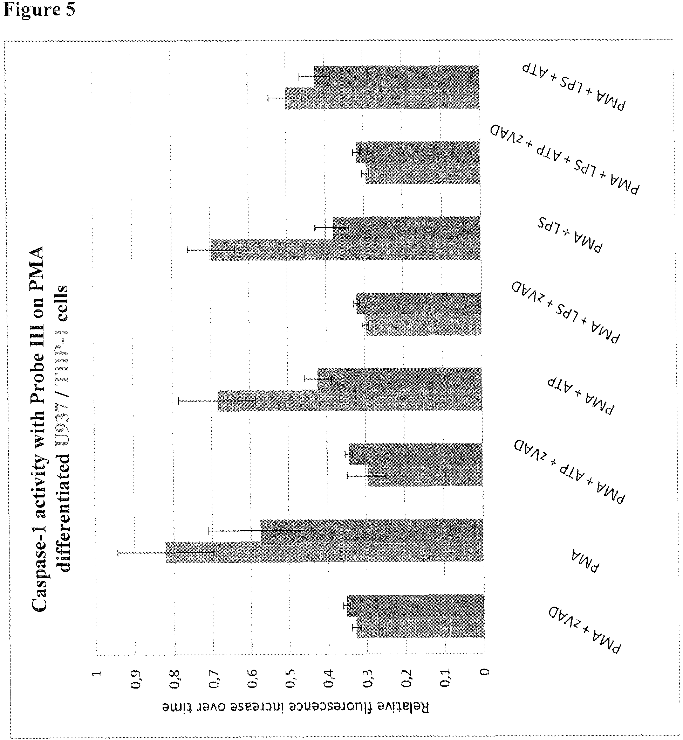

Figure 5: Cellular assay with Probe III; PMA=Phorbol-12-myristate-13-acetate; LPS=Lipopolysaccharide; zVAD=carbobenzoxy-valyl-alanyl-asparty-[O-methyl]-fluoromethylketone

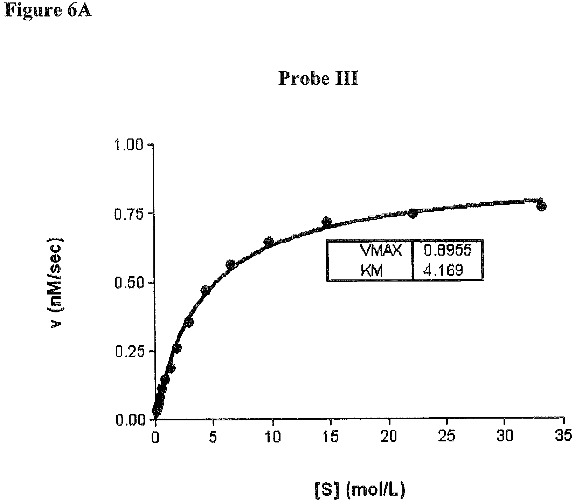

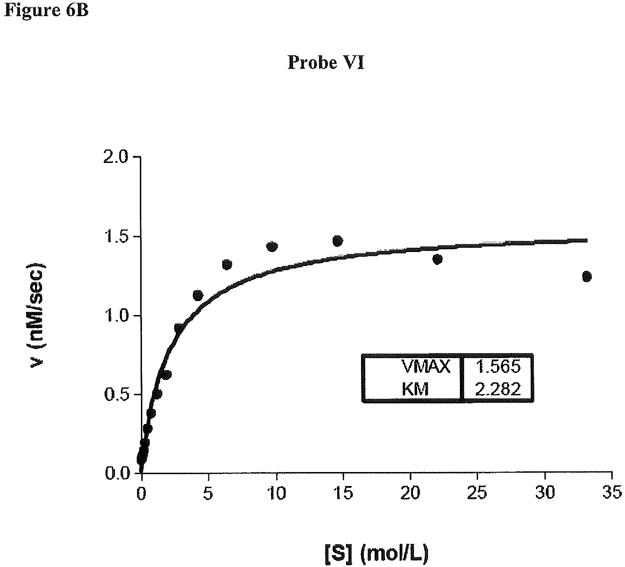

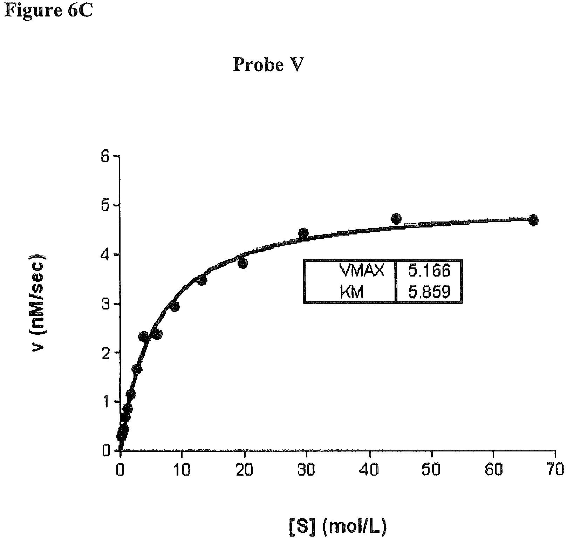

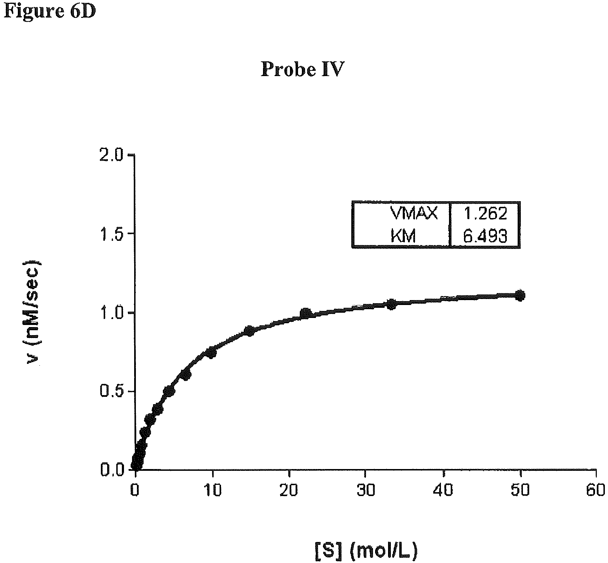

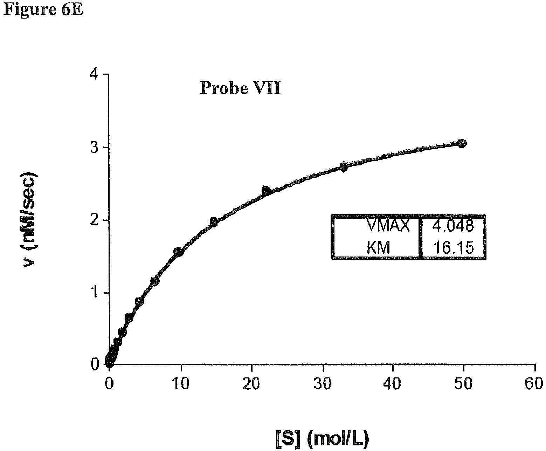

Figure 6A-E: Kinetic characterization of probes II, VI, V, IV, and VII.

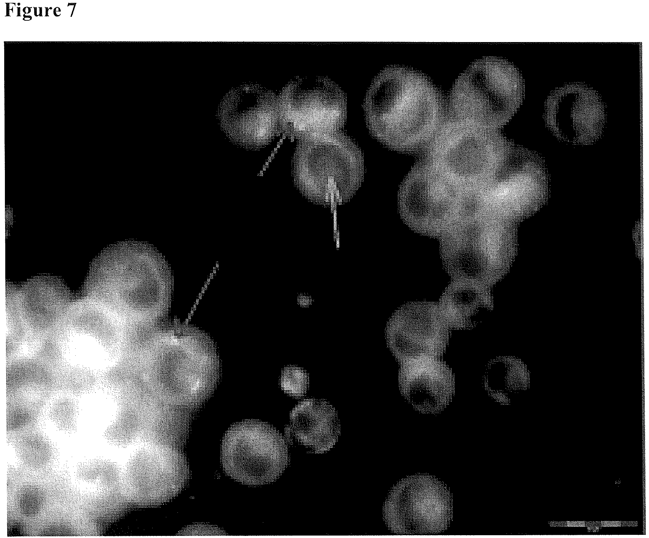

Figure 7: Cell permeability assay.

Rozman-Pungercar, J., Kopitar Jerala, N., Bogyo, M., Turk,D., Vasiljeva, O., Stefe, I., Vandenabeele, P., Brömme, D., Puizdar, V., Fonovic, M., Trstenjak-Prebanda, M., Dolenc, I., Turk, V., Turk, B. (2003) Inhibition of papain-like cysteine proteases and legumain by "caspase-specific" inhibitors: when reaction mechanism is more important than specificity. Cell Death Diff. 10, 881-888.
Kindermann M, Roschitzki-Voser H, Caglic D, Repnik U, Miniejew C, Mittl PR, Kosec G, Grütter MG, Turk B, Wendt KU. (2010) Selective and sensitive monitoring of caspase-1 activity by a novel bioluminescent activity-based probe. Chemistry & Biology. 17, 999-100.

### Examples

### Abbreviations:

DMF = dimethylformamide
DMSO = dimethylsulfoxide
DCM = dichloromethane
equiv. = equivalents
sat. = saturated
THF = tetrahydrofuran
DIPEA = diisopropylethyl amine
HOAt = 1-Hydroxy-7-azabenzotriazole
HATU = *O*-(7-Azabenzotriazol-1-yl)-*N*,*N*,*N'*,*N'*-tetramethyluronium hexafluorophosphate,
NHS = N-hydroxysuccinimidyl ester

### 1. General Information and Material

Unless otherwise noted, all reagents were purchased from commercial suppliers and used without further purification. All solvents used were HPLC grade. Reactions were analyzed by thin-layer chromatography on Merck 50x100 mm silica gel 60 aluminium sheets with fluorescent indicator or LC-MS. Column chromatography was carried out with Merck silica gel (0.040-0.063 mm). Reverse-phase HPLC was performed on a C-18 column Sun Fire (50x100 mm, Waters) or XBridge^{™}Prep C-18, (5 µm, 10x100 mm, Waters). LC-MS data were acquired using the HP-Agilent 1100 MSD system. All HRMS spectra were recorded on a LTQ-Orbitrap Elite (ThermoElectron) mass spectrometer equipped with a C-18 column (3.5 mm, 50 x 3 mm (#233)) using a 0.5 mL/min CH₃CN/water gradient (0.1% TFA) and detection by UV (eLambda PDA (waters)) and MS (ESI+) at 25 °C.

NMR spectra were recorded on a Bruker AVANCE 500 spectrometer operating at 500 MHz for ¹H equipped with a 5mm TCI cryo probe head or on a Bruker AVANCE 600 spectrometer operating at 600 MHz for ¹H equipped with a 5mm standard TXI probe head. For a complete assignment, usually proton, carbon, DQF-COSY, ROESY, edited-HSQC and HMBC spectra have been acquired. All experiments were carried out at 300K in DMSO-*d₆*. NMR spectra were referenced to the solvent resonance signal (DMSO-*d₆* : ¹H δₚₚₘ 2.50, ¹³C δₚₚₘ 39.5). Homonuclear experiments, DQF-COSY and ROESY, were performed with a spectral width according to the 1D spectra. Spectra were recorded with 1024 increments in t₁ and 4096 complex data points in t₂. For each t₁ value 2 - 8 transients were averaged (depending on the amount of sample and the spectrometer which has been used). For the ROESY spectrum a mixing time of 150 ms was used. For HSQC spectra 1024 increments (2 to 8 scans) with 2048 complex data points in t₂ were collected using a sweep width of up to 10 ppm in the proton and 160 ppm in the carbon dimension. The HMBC spectra were acquired with a sweep width according to the 1D spectra in the proton and 200 ppm in the carbon dimension. A total of 4 - 16 transients were averaged for each of 1024 increments in t₁, and 4096 complex points in t₂ were recorded.

### 2. Scheme for the synthesis of Probe I (19)

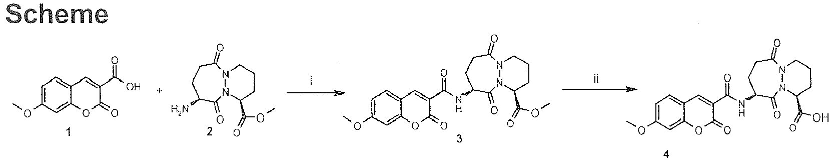

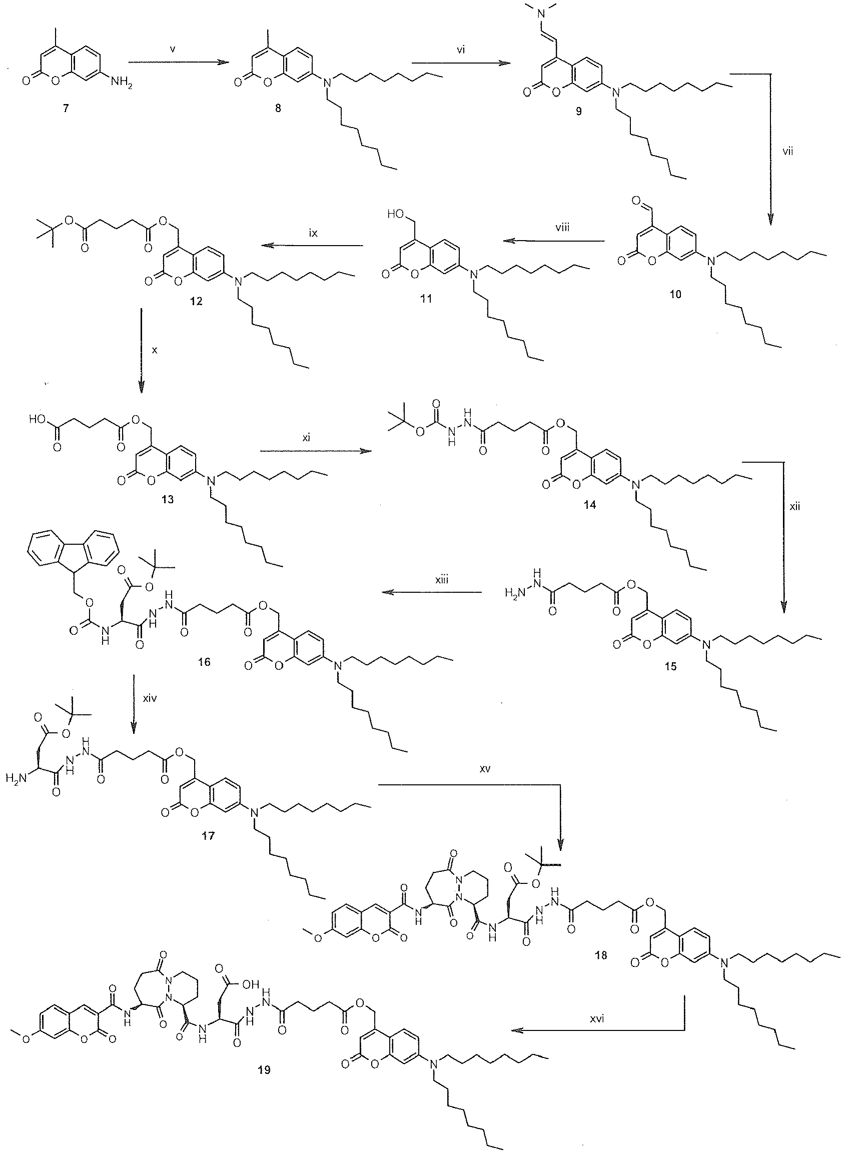

**Reagents and conditions**: (i) HATU, DIPEA, DMF, 1 h, RT, (ii) LiOH / THF:Water:Methanol (2:1:1) RT, 4 h, (iii)-(iv) *t*-BuOH, Et₃N, DMAP, *N-*hydroxy succinimide, Toluene, 110 °C, O.N., (v) 1-bromooctane, DIPEA, Nal, DMF, 120 °C, 4 d, (vi) *N*,*N*-dimethylformamide dimethylacetal, DMF, 140 °C, 16 h, (vii) NalO₄ H₂O, THF, 3 h, (viii) NaBH₄, MeOH, 3h, (ix) **6**, EDC.HCL, DMAP, DMF, 1.5 h, (x) 50% TFA/DCM, 15 min (xi) BocNHNH₂, HATU, DIPEA, DMF, 30 min, (xii) 50% TFA/DCM, 15 min (xiii) Fmoc-Asp(O*^{t}*Bu)-OH, HATU, DIPEA, DMF, RT, 1h, (xiv) 20% piperidine in DMF, 1 h (xv) **Compound 4**, HATU, DIPEA, DMF, RT, 2h, (xvi) 50% TFA/DCM, 15 min.

### 3. Synthesis of Probe I(19)

### a) Synthesis of Compound 4 in the Scheme of Example 2

Commercially available 7-Methoxycoumarin-3-carboxylic acid (**1**, 2.0 equiv) and HATU (1.9 equiv) were weighed in an oven dry round bottom flask. Minimum amount of dry DMF was added and the mixture was stirred with a Teflon coated stirring bar under argon atmosphere at room temperature for 10-15 min to affect total dissolution. DIPEA (4.0 equiv) was then added to the stirred solution for a thorough mixing. Thereafter, 6,7-bicycloamine scaffold (**2**, 1.0 equiv) was added to the reaction mixture under argon atmosphere and stirred for 1 h at room temperature. The progress of reaction was monitored by LC-MS and TLC. After a productive coupling and complete consumption of limiting starting material (**2**), the reaction was quenched by the addition of 100 µL of TFA. The desired product **3** was then purified using a reverse-phase HPLC. The compound was then freeze-dried under vacuam and ready to use for the next step. Coupled product (**3**): Retention time 1.24 min (RP LCMS and TOP.M method)
MS (ESI pos.) calculated for C₂₂H₂₃N₃O₈: 457.0, Found 458.1 [M+H]⁺ Freeze-dried compound **3** (1.0 equiv) was then transferred to a round bottom flask equipped with a magnetic stirrer and subjected under a productive mild ester hydrolysis condition using LiOH (5.0 equiv) and a minimum volume of THF:Water:Methanol (2:1:1) solvent system at room temperature for 4 h. After complete hydrolysis (reaction monitored by LC-MS), the reaction was quenched by addition of 100 µL of TFA. The reaction mixture was evaporated to dryness and purified by reverse-phase HPLC. The chirally pure product (**4**), was freeze dried and used for further coupling. A complete analytical characterization was performed for **Compound 4** and the analysis report is attached at the end of this supporting information.
**¹³C-NMR** (**125 MHz, d₆-DMSO**): δ = 171.08, 171.01, 169.23, 164.65, 160.85, 160.62, 156.32, 148.40, 131.72, 113.88, 113.74, 112.06, 100.30, 56.27, 52.26, 48.75, 40.46, 30.56, 29.33, 24.54, 19.89
**¹H-NMR (600 d₆-DMSO)**: δ = 13.40 (s, 1 H), 9.37 (d, 1 H), 8.88 (s, 1 H), 7.93 (d, 1 H), 7.13 (d, 1 H), 7.05 (dd, 1 H), 5.26 (dd, 1 H), 4.87 (m, 1 H), 4.37 (m, 1 H), 3.91 (s, 3H), 3.39 (m, 1 H), 3.01 (m, 1 H), 2.66 (m, 1 H), 2.19 (m, 1 H), 2.11 (m, 1 H), 1.88 (m, 1 H), 1.87 (m, 1 H), 1.68 (m, 1 H), 1.50 (m, 1 H)
MS (ESI pos.) calculated for C₂₁H₂₁N₃O₈: 443.0, Found 444.1 [M+H]⁺

### b) Compound 6 (Pentanedioic acid mono-tert butyl ester) used in step (ix) is commercially available.

### c) Synthesis of N,N-dioctylamino-4-hydroxymethyl-2-oxo-2H-chromen (11)

1) 7-*N*,*N*-Dioctylamino-4-methyl-2-oxo-2*H*-chromen (**8**) 7-Amino-4-methyl-2-oxo-2*H*-chromen (**7**, 5.4 g, 30.8 mmol), 1-bromooctane (26.6 mL, 154.1 mmol), DIPEA (29 mL, 169.5 mmol) and sodium iodide (532 mg, 3.5 mmol) in DMF (50 mL) were heated to 120 °C for 4 d with stirring under argon atmosphere. The black mixture is allowed to cool to room temperature and volatiles were distilled off. The residue obtained is extracted with EtOAc:water (1:1, 100 mL), the organic layer separated, washed with water (50 mL) and evaporated under reduced pressure. The resulting oil (18.0 g) was distillated under high vacuum for 1 h to afford a residue that was purified by chromatography on a silica column with (i) cyclohexane:EtOAc 1:0 and (ii) 1:4. The fractions containing the compound were combined, evaporated under reduced pressure and again subjected to chromatography on silica gel with cyclohexane:EtOAc 9:1 as eluant.
Yield: 9.8 g (79.6%) oil, R_{f}: cyclohexane:EtOAc 9:1 = 0.28, t_{R} 100% MeOH = 5.4 min
¹H NMR (400 MHz, CDCl₃) δ = 7.37 (d, *J*=9.0, 1 H), 6.54 (dd, *J*=9.0, 2.6, 1 H), 6.46 (d, *J*=2.5, 1 H), 5.93 (d, *J*=1.0, 1 H), 3.37 - 3.22 (m, 4H), 2.33 (d, *J*=1.0, 3H), 1.66 - 1.53 (m, 4H), 1.38 - 1.17 (m, 20H), 0.89 (t, *J*=6.9, 6H).
MS (ESI pos.) calculated for C₂₆H₃₉NO₂ : 399.31, Found 400.5 [M+H]⁺

### 2) 2-(7-N,N-Dioctylamino-2-oxo-2H-chromen-4-yl)-N,N-dimethylaminoethen (9)

A solution of 7-*N*,*N*-dioctylamino-4-methyl-2-oxo-2*H*-chromen (**8**, 9.6 g, 24 mmol) in *N*,*N*-dimethylformamide dimethylacetal (6.4 mL, 48 mmol) and anhydrous DMF (18 mL) was heated to 140 °C for 16 h with stirring under argon atmosphere. The black solution is allowed to cool to room temperature and volatiles were distilled off in high vacuum. The crude compound was purified by silica gel column chromatography with 1. cyclohexane:EtOAc 9:1 and 2. DCM:EtOAc 4:1.
Yield: 7.26 g (66.6%) solid, R_{f} : cyclohexane:EtOAc 4:1 = 0.10, t_{R} 100% MeOH = 5.7 min
¹H NMR (400 MHz, CDCl₃) δ = 7.53 (d, *J*=9.0, 1 H), 7.23 (d, *J*=12.9, 1 H), 6.48 (m, 2H), 5.86 (s, 1 H), 5.22 (d, *J*=13.0, 1 H), 3.29 (t, *J*=6.0, 4H), 2.99 (s, 6H), 1.68 - 1.50 (m, 4H), 1.29 (m, 20H), 0.88 (t, J=6.8, 6H).
MS (ESI pos.) calculated for C₂₉H₄₆N₂O₂: 454.65, Found 455.4 [M+H]⁺ 3) 7-*N*,*N*-Dioctylamino-4-formyl-2-oxo-2*H*-chromen (**10**) 2-(7-*N*,*N*-Dioctylamino-4-yl-2-oxo-2*H*-chromen-4-yl)-*N*,*N-*dimethylaminoethen (**9**, 5.76 g, 12.7 mmol) was dissolved in THF (150 mL). Water (150 mL) and sodium periodate (8.56 g, 40 mmol) were subsequently added. After 3 h with vigorous stirring the mixture was diluted with EtOAc (200 mL), filtered, the organic phase was separated, washed with saturated sodium hydrogencarbonate solution (2 x 100 mL), dried (Na₂SO₄), filtered and evaporated under reduced pressure. The residue obtained was purified by silica gel column chromatography with 1. cyclohexane:EtOAc 1:0, 2. 9:1 and 3. 4:1.
Yield: 2.85 g (54.4%), R_{f} :cyclohexane: EtOAc 4:1 = 0.77, t_{R} 100% MeOH = 3.4 min
¹H NMR (400 MHz, CDCl₃) δ = 10.03 (s, 1H), 8.30 (d, *J*=9.2, 1H), 6.60 (dd, *J*=9.2, 2.6, 1H), 6.49 (d, *J*=2.5, 1 H), 6.45 (s, 1 H), 3.46 - 3.16 (m, 4H), 1.68 - 1.55 (m, 4H), 1.38 - 1.20 (m, 20H), 0.89 (t, *J*=6.8, 6H). MS (ESI pos.) calculated for C₂₉H₃₉NO₃ : 413.29, Found 414.1 [M+H]⁺

### 4) 7-N,N-Dioctylamino-4-hydroxymethyl-2-oxo-2H-chromen (11)

7-*N*,*N*-Dioctylamino-4-formyl-2-oxo-2*H*-chromen (**10**, 2.7 g, 6.53 mmol) in MeOH (75 mL) is treated with sodium borohydride (2.26 g, 59.8 mmol) in small portions with stirring. After 3 h the reaction was concentrated under reduced pressure, neutralized with 1 N HCl and extracted three times with EtOAc (3 x 50 mL). The combined organic phases were dried (Na₂SO₄), filtered and evaporated under reduced pressure. The residue obtained was purified by silica gel column chromatography using 1. cyclohexane:EtOAc 9:1, 2. 4:1 and 3. 7:3.
Yield: 2.53 g (93.2%) solid, R_{f} : cyclohexane:EtOAc 4:1 = 0.10, t_{R} 100% MeOH = 3.5 min

**¹H NMR(600 MHz, d₆-DMSO)** δ = 7.41 (d, 1 H), 6.61 (dd, 1 H), 6.46 (d, 1 H), 6.06 (s, 1 H), 5.50 (broad, 1 H), 4.65 (s, 2H), 3.32 (t, 4H), 1.51 (m, 4H), 1.32-1.20 (m, 20 H), 0.85 (t, 6H)

**¹³C NMR (125 MHz, d₆-DMSO):** δ = 161.11, 156.79, 155.50, 150.54, 124.92, 108.56, 105.63, 103.85, 96.81, 59.00, 50.10, 31.21, 28.83, 28.69, 26.68, 26.27, 22.05, 13.91

**MS** (ESI pos.) calculated for C₂₆H₄₁NO₃: 415.61, Found 416.3 [M+H]⁺

### d) Synthesis of Probe I (19)

In an oven dried round bottom flask, **Compound 11** (1.0 equiv), Pentanedioic acid mono-tert butyl ester (**6**, 1.1 equiv), EDC HCl (1.2 equiv) and DMAP (0.3 equiv) were dissolved in minimum amount of DMF. The mixture was stirred at 20 °C for 1.5 h. Analytical LCMS indicated complete consumption of coumarin and formation of ester (**12**). Compound **12** (MS (ESI pos.) calculated for C₃₅H₅₅NO₆: 585.0, found 586.3 [M+H]⁺, retention time = 2.869 min) was then purified via preparative HPLC and the pure fractions were collected and evaporated to dryness. The pure **12** was then treated with 50% TFA/DCM to get compound **13**. The HPLC purified **13** (MS (ESI pos.) calculated for C₃₁H₄₇NO₆: 529.0, found 530.2 [M+H]⁺, retention time = 2.441 min (RP LCMS and modified TOP.M method for long chain hydrocarbon and mass range (Mw) 80-1500 with 4.0 min extra run at 95.0 % AcCN)) was then used for the next step.

In an oven dried round bottom flask, **13** (1.0 equiv) and HATU (0.9 equiv) were weighed. Minimum amount of dry DMF was added and the mixture was stirred with a Teflon coated stirring bar under argon atmosphere at room temperature for 10-15 min to affect total dissolution. DIPEA (2.0 equiv) was then added to the stirred solution for a thorough mixing. Thereafter, mono boc-protected hydrazine (1.2 equiv) was added to the reaction mixture under argon atmosphere for 30 min. Compound **14** was formed rapidly and reaction was quenched adding 50 µL TFA and purified via reverse phase HPLC. To access the installed hydrazine motif, the tert-butyl group required to be removed. Compound **14** (MS (ESI pos.) calculated for C₃₆H₅₇N₃O₇: 643.0, found 644.3 [M+H]⁺, retention time = 2.478 min (RP LCMS and modified TOP.M method for long chain hydrocarbon and mass range (Mw) 80-1500 with 4.0 min extra run at 95.0 % AcCN)) was thereafter treated with 50% TFA/DCM to furnish compound **15**. The HPLC purified **15** (MS (ESI pos.) calculated for C₃₁H₄₉N₃O₅: 543.0, found 544.3 [M+H]⁺, retention time = 2.012 min (RP LCMS and modified TOP.M method for long chain hydrocarbon and mass range (Mw) 80-1500 with 4.0 min extra run at 95.0 % AcCN)) was then used for amino acid coupling.

Fmoc-Asp(O*t*Bu)-OH (1.2 equiv) and HATU (1.1 equiv) were taken in a dry round bottom flask. Then minimum volume of dry DMF was added stirred for 15 min. DIPEA (2.0 equiv) was added and mixed thoroughly. Compound **15** (1.0 equiv) was added to the mixture and stirred for 1 h until the completion of the reaction. The reaction was then quenched with 15 µL of TFA and pure product **16** (MS (ESI pos.) calculated for C₅₄H₇₂N₄O₁₀: 936.2, found 937.7 [M+H]⁺, retention time = 2.710 min (RP LCMS and modified TOP.M method for long chain hydrocarbon and mass range (Mw) 80-1500 with 4.0 min extra run at 95.0 % AcCN)) was obtained after HPLC purification. The purified **16** was subjected to 20% piperidine in DMF for the removal of fmoc-group. After fmoc removal, compound **17** (MS (ESI pos.) calculated for C₃₉H₆₂N₄O₈: 714.0, found 715.3 [M+H]⁺, retention time = 1.822 min (RP LCMS and modified TOP.M method for long chain hydrocarbon and mass range (Mw) 80-1500 with 4.0 min extra run at 95.0 % AcCN)) was then ready for the final step coupling reaction.

To a stirred solution of **Compound 4** (1.2 equiv) and HATU (1.1 equiv) in DMF, DIPEA (2.0 equiv) was added and mixed thoroughly. Compound **17** (1.0 equiv) was taken into 1 mL (500 µL x 2) DMF and added to the mixture at room temperature and stirred for 2 h. After complete consumption of **17**, the new product **18** was formed and was isolated by HPLC. The pure **18** (MS (ESI pos.) calculated for C₆₀H₈₁N₇O₁₅: 1140.3, found 1141.7 [M+H]⁺, retention time = 2.394 min (RP LCMS and modified TOP.M method for long chain hydrocarbon and mass range (Mw) 80-1500 with 4.0 min extra run at 95.0 % AcCN)) was then treated with 50% TFA/DCM for the removal of tert-butyl group on Asp to get the desired product **19**. The final compound was further purified via preparative HPLC. A complete analytical characterization was performed for **Probe I (19)** and the analysis report is attached at the end of this supporting information. The analysis confirmed the structure of **Probe I (19).**

**¹³C-NMR (125 MHz, d₆-DMSO):** δ = 172.10, 171.49, 171.21, 170.50, 169.76, 169.41, 169.18, 164.66, 160.90, 160.60 (2 C), 156.34, 155.67, 150.87, 150.59, 148.42, 131.75, 125.38, 113.91, 113.77, 112.09, 108.86, 105.22, 105.01, 100.30, 96.90, 61.09, 56.29, 52.00, 50.11, 48.82, 48.33, 40.58, 35.99, 32.40, 32.09, 31.19, 30.34, 29.75, 28.81, 28.67, 26.67, 26.24, 25.39, 22.05, 20.30, 19.03, 13.92

**¹H-NMR (600 MHz, d₆-DMSO):** δ **=** 9.84 (s, 1 H), 9.83 (s, 1 H), 9.36 (d, 1 H), 8.89 (s, 1 H), 8.49 (d, 1 H), 7.94 (d, 1 H), 7.45 (d, 1 H), 7.14 (d, 1 H), 7.06 (dd, 1 H), 6.67 (dd, 1 H), 6.50 (d, 1 H), 5.97 (t, 1 H), 5.28 (s, 2H), 5.18 (dd, 1 H), 4.85 (m, 1 H), 4.59 (m, 1 H), 4.32 (m, 1 H), 3.91 (s, 3H), 3.35 (t, 4H), 3.34 (m, 1 H), 2.96 (m, 1 H), 2.73 (dd, 1 H), 2.61 (m, 1 H), 2.57 (dd, 1 H), 2.51 (t, 2H), 2.17 (t, 2H), 2.13 (m, 1 H), 2.06 (m, 1 H), 1.86 (m, 1H), 1.81 (m, 2H), 1.81 (m, 1 H), 1.65 (m, 1 H), 1.54 (m, 1 H), 1.52 (m, 4H), 1.30 - 1.20 (m, 20H), 0.85 (t, 6H)

**HRMS:** The sample was ionized by ESI. The monoisotopic mass was calculated from pseudo molecular ion [M+H]+ and is in agreement with the theoretically expected mass with a deviation of less than 1 ppm, confirming an elemental composition of C₅₆H₇₃N₇O₁₅.
M_{monoisotopic, experimental}: **1083.51562** Da; _{Mmonoisotopic, theoretical}: **1083.51646** Da

### 4. Scheme for the synthesis of Probe II

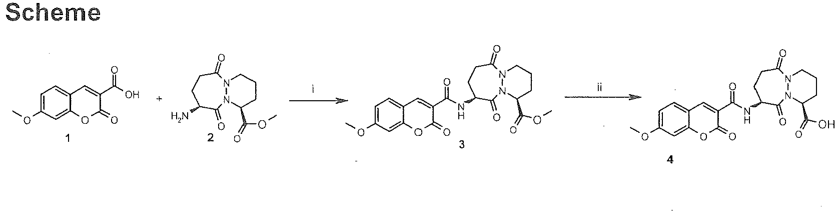

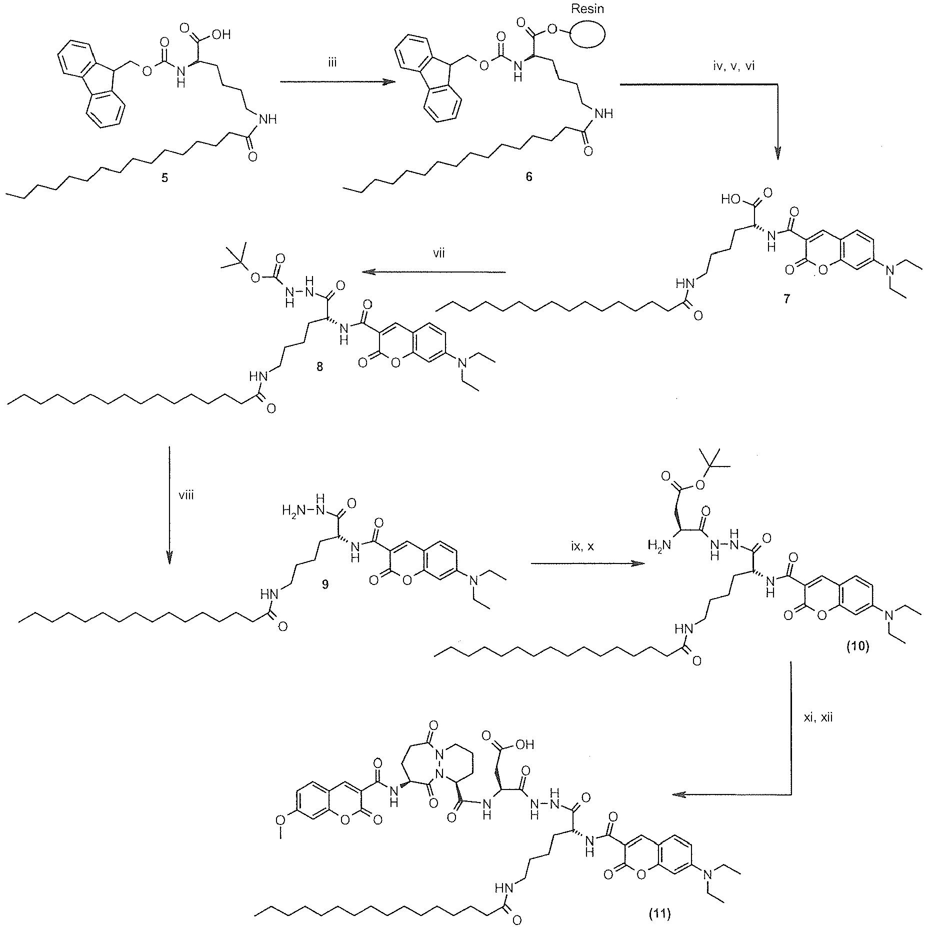

**Reagents and conditions:** (i) HATU, DIPEA, DMF, 1 h, RT, (ii) LiOH / THF:Water:Methanol (2:1:1) RT, 4 h, (iii) 2-chlorotrityl resin, DIPEA, DCM, over night, (iv) 20% piperidine in DMF, 1 h, (v) 7-diethylaminocoumarin-3-carboxylic acid, HATU, DIPEA, DMF, 1.5 h, (vi) 2% TFA/DCM, 15 min, (vii) BocNHNH₂, HATU, DIPEA, DMF, 30 min, (viii) 50% TFA/DCM, 15 min, (ix) Fmoc-Asp(O*^{t}*Bu)-OH, HATU, DIPEA, DMF, RT, 1 h, (x) 20% piperidine in DMF, 1 h (xi) (4), HATU, DIPEA, DMF, RT, 2h, (xii) 50% TFA/DCM, 15 min.

### 5. Synthesis of Compound II (11)

### a) Synthesis of Compound was carried out according compound 4 of probe I using Starting material 1

### b) Synthesis of Probe II (11)

The synthesis of (**11**) was a combination between solid-support and solution phase chemistry. Commercially available 2-chlorotrityl resin (1.0 equiv) was used as solid-support (Novabiochem, capacity: 1.4 mmol/g) and transferred to a syringe and thoroughly rinsed with HPLC grade DCM. The resin was then charged with Fmoc-Lys(palmitoyl)-OH (**5**, 2.0 equiv) and DIPEA (4.0 equiv) in dry DCM. The solution was then stirred at room temperature over night. The resin was filtered and washed with DCM (3 x 2.0 mL, 5.0 min for each cycle) and DMF (3 x 2.0 mL, 5.0 min for each cycle). The resin bound amino acid (**6**) was then treated with 20% piperidine in DMF and stirred at room temperature for 1 h for complete removal of fmoc group (detected via Kaiser test kit). It was then washed thoroughly with DMF (5 x 2.0 mL, 5.0 min for each cycle). The resin bound N-deprotected amino acid was then treated with 7-diethylaminocoumarin-3-carboxylic acid (2.0 equiv), HATU (1.9 equiv) and DIPEA (4.0 equiv) in dry DMF. The mixture was then stirred for 1.5 h for a productive coupling. It was then filtered and washed with DMF. The resin bound amino acid coupled with diethylaminocoumarin moiety was then treated with 2% TFA/DCM for 15 min (x 2 times) for a complete resin cleavage to affect. The filtrate was collected along with successive washing solvent (2 x with 2% TFA/DCM and 3 x 2.0 mL DCM) and evaporated to dryness, furnished compound **7** (MS (ESI pos.) calculated for C₃₆H₅₇N₃O₆: 627.0, found 628.3 [M+H]⁺, retention time = 3.638 min (RP LCMS and TOP_LANG.M method).

In an oven dried round bottom flask, **7** (1.0 equiv) and HATU (0.9 equiv) were transferred. Minimum amount of dry DMF was added and the mixture was stirred with a Teflon coated stirring bar under argon atmosphere at room temperature for 10-15 min to affect total dissolution. DIPEA (2.0 equiv) was then added to the stirred solution for a thorough mixing. Thereafter, mono boc-protected hydrazine (1.2 equiv) was added to the reaction mixture under argon atmosphere for 30 min. Compound **8** was formed rapidly and reaction was quenched adding 50 µL TFA and purified via reverse phase HPLC. To access the installed hydrazine motif, the tert-butyl group required to be removed. Compound **8** (MS (ESI pos.) calculated for C₄₁H₆₇N₅O₇: 741.0, found 742.4 [M+H]⁺, retention time = 3.897 min (RP LCMS and TOP_LANG.M method) was thereafter treated with 50% TFA/DCM to furnish compound **9.** The HPLC purified **9** (MS (ESI pos.) calculated for C₃₆H₅₉N₅O₅: 641.0, found 642.4 [M+H]⁺, retention time = 3.218 min (RP LCMS and TOP_LANG.M method) was then used for amino acid coupling.

Fmoc-Asp(O^{t}Bu)-OH (1.2 equiv) and HATU (1.1 equiv) were taken in a dry round bottom flask. Then minimum volume of dry DMF was added stirred for 15 min. DIPEA (2.0 equiv) was added and mixed thoroughly. Compound **9** (1.0 equiv) was added to the mixture and stirred for 1 h until the completion of the reaction. The reaction was then quenched with 15 µL of TFA and pure product was obtained after HPLC purification. The purified product was then subjected to 20% piperidine in DMF for the removal of fmoc-group. After fmoc removal, compound **10** was characterized analytically before the final step coupling reaction.

### Compound 10

**¹H-NMR (600 MHz, d6-DMSO):** δ = 10.46 (s, 1 H), 10.44 (s, 1 H), 9.01 (d, 1 H), 8.65 (s, 1 H), 8.27 (d, 3H), 7.69 (d, 1 H), 6.82 (dd, 1 H), 6.63 (d, 1 H), 4.62 (m, 1 H), 4.11 (m, 1 H), 3.49 (q, 4H), 2.99 (m, 2H), 2.87 (dd, 1 H), 2.77 (dd, 1 H), 1.99 (t, 2H), 1.72 (m, 1 H), 1.63 (m, 1 H), 1.45-1.32 (m, 6H), 1.43 (s, 9H), 1.28-1.11 (m, 24H), 1.14 (t, 6H), 0.85 (t, 3H)

**HRMS:** The sample was ionized by ESI. The monoisotopic mass was calculated from pseudo molecular ion [M+H]+ and is in agreement with the theoretically expected mass with a deviation of less than 1 ppm, confirming an elemental composition of C₄₄H₇₂N₆O₈.
M_{monoisotopic, experimental}: **812.54136** Da; _{Mmonoisotopic, theoretical}: **812.54116** Da

To a stirred solution of **(4)** (1.2 equiv) and HATU (1.1 equiv) in DMF, DIPEA (2.0 equiv) was added and mixed thoroughly. Compound **10** (1.0 equiv) was taken into 1 mL (500 µL x 2) DMF and added to the mixture at room temperature and stirred for 2 h. After complete consumption of **10,** the new coupled product was formed and was isolated by HPLC. The HPLC pure coupled product of (**4**) and (**10**) was then treated with 50% TFA/DCM for the removal of *tert*-butyl group on Asp to get the desired product **11**. The final compound was further purified via preparative HPLC. A complete analytical characterization was performed for **11** and the analysis report is attached at the end of this supporting information. The analysis confirmed the structure of **11**.

### Probe II

**¹H-NMR (600 MHz, d₆-DMSO):** δ = 10.19 (s, 1 H), 9.96 (s, 1 H), 9.37 (d, 1 H), 9.00 (d, 1 H), 8.89 (s, 1 H), 8.66 (s, 1 H), 8.51 (d, 1 H), 7.94 (d, 1 H), 7.69 (d, 1 H), 7.67 (t, 1 H), 7.14 (d, 1 H), 7.06 (dd, 1 H), 6.81 (dd, 1 H), 6.63 (d, 1 H), 5.18 (dd, 1 H), 4.85 (m, 1 H), 4.59 (m, 1 H), 4.57 (m, 1 H), 4.33 (m, 1 H), 3.90 (s, 3H), 3.48 (q, 4H), 3.34 (m, 1 H), 2.99 (dt, 2H), 2.96 (m, 1 H), 2.74 (dd, 1 H), 2.62 (m, 1 H), 2.58 (dd, 1 H), 2.13 (m, 1 H), 2.06 (m, 1 H), 1.99 (t, 2H), 1.86 (m, 1 H), 1.81 (m, 1 H), 1.72 (m, 1 H), 1.65 (m, 1 H), 1.63 (m, 1 H), 1.55 (m, 1 H), 1.42 (m, 2H), 1.39 (m, 2H), 1.32 (m, 2H), 1.30-1.20 (m, 24H), 1.14 (t, 6H),

**HRMS:** The sample was ionized by ESI. The monoisotopic mass was calculated from pseudo molecular ion [M+H]+ and is in agreement with the theoretically expected mass with a deviation of less than 1 ppm, confirming an elemental composition of C₆₁H₈₃N₉O₁₅.
M_{monoisotopic, experimental} **1181.60155** Da; _{Mmonoisotopic}, _{theoretical}: **1181.60086** Da

### 6. Scheme for the synthesis of Probe III, Probe IV, & Probe V

### Scheme

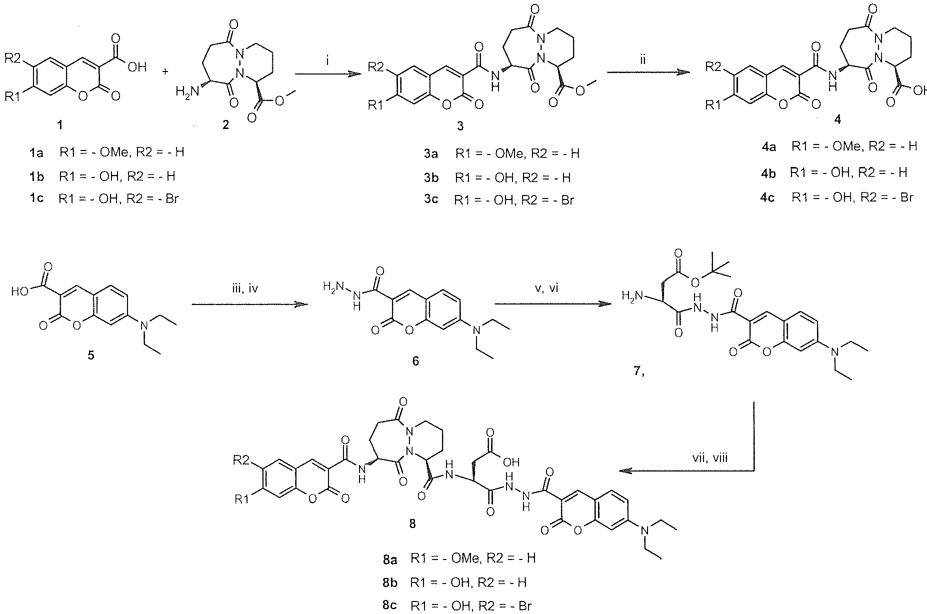

**Reagents and conditions:** (i) HATU, DIPEA, DMF, 1 h, RT, (ii) LiOH / THF:Water:Methanol (2:1:1) RT, 4 h, (iii) BocNHNH₂, HATU, DIPEA, DMF, 30 min, (iv) 50% TFA/DCM, 15 min, (v) Fmoc-Asp(O^{t}Bu)-OH, HATU, DIPEA, DMF, RT, 1h, (vi) 20% piperidine in DMF, 1 h (vii) **4,** HATU, DIPEA, DMF, RT, 2h, (viii) 50% TFA/DCM, 15 min.

### 7. Synthesis of 8

### a) Synthesis of 4a, 4b,and 4c was carried out analogously as the synthesis of compound 4 of probe I using Starting material 1a, 1b, 1c.

The desired product 3 was then purified using a reverse-phase HPLC. The compound was then freeze-dried under vacuum and ready to use for the next step.

### Coupled product (3a): Retention time 1.24 min (RP LCMS and TOP.M method)

MS (ESI pos.) calculated for C₂₂H₂₃N₃O₈ : 457.0, Found 458.1 [M+H]⁺ Coupled product (**3b**): Retention time 1.106 min (RP LCMS and TOP.M method)
MS (ESI pos.) calculated for C₂₁H₂₁N₃O₈ : 443.0, Found 444.1 [M+H]⁺

### Coupled product (3c): Retention time 1.215 min (RP LCMS and TOP.M method)

MS (ESI pos.) calculated for C₂₁H₂₀BrN₃O₈ : 522.4, Found 523.7 [M+H]⁺

### 4a

**¹³C-NMR (125 MHz, d₆-DMSO):** δ = 171.08, 171.01, 169.23, 164.65, 160.85, 160.62, 156.32, 148.40, 131.72, 113.88, 113.74, 112.06, 100.30, 56.27, 52.26, 48.75, 40.46, 30.56, 29.33, 24.54, 19.89
**¹H-NMR (600 MHz, d₆-DMSO):** δ = 13.40 (s, 1 H), 9.37 (d, 1 H), 8.88 (s, 1 H), 7.93 (d, 1 H), 7.13 (d, 1 H), 7.05 (dd, 1 H), 5.26 (dd, 1 H), 4.87 (m, 1 H), 4.37 (m, 1 H), 3.91 (s, 3H), 3.39 (m, 1 H), 3.01 (m, 1 H), 2.66 (m, 1 H), 2.19 (m, 1 H), 2.11 (m, 1 H), 1.88 (m, 1 H), 1.87 (m, 1 H), 1.68 (m, 1 H), 1.50 (m, 1 H)
**MS** (ESI pos.) calculated for C₂₁H₂₁N₃O₈: 443.0, Found 444.1 [M+H]⁺

### 4b

**¹³ (125 MHz, d₆-DMSO):** δ = 171.11, 171.05, 169.27, 163.91, 161.01, 160.80, 156.44, 148.60, 132.21, 114.42, 112.75, 111.08, 101.82, 52.27, 48.73, 40.46, 30.61, 29.35, 24.56, 19.93
**¹H-NMR (600 MHz, d₆-DMSO): δ** = 13.41 (s, 1H), 11.12 (s, 1H), 9.36 (d, 1 H), 8.83 (s, 1 H), 7.84 (d, 1 H), 6.89 (dd, 1 H), 6.82 (d, 1 H), 5.26 (dd, 1 H), 4.86 (m, 1 H), 4.36 (m, 1 H), 3.38 (m, 1 H), 3.01 (m, 1 H), 2.65 (m, 1 H), 2.18 (m, 1 H), 2.10 (m, 1 H), 1.87 (m, 1 H), 1.67 (m, 1 H), 1.49 (m, 1 H)
**MS** (ESI pos.) calculated for C₂₀H₁₉N₃O₈: 429.0, Found 430.0 [M+H]⁺

### 4c

**¹H-NMR (600 MHz, d₆-DMSO):** δ = 13.42 (1 H, s), 12.00 (s, 1 H), 9.33 (d, 1 H), 8.82 (s, 1 H), 8.27 (s, 1 H), 6.94 (s, 1 H), 5.25 (dd, 1 H), 4.86 (m, 1 H), 4.33 (m, 1 H), 3.37 (m, 1 H), 3.00 (m, 1 H), 2.65 (m, 1 H), 2.18 (m, 1H), 2.10 (m, 1 H), 1.87 (m, 1 H), 1.67 (m, 1 H), 1.49 (m, 1 H)

**HRMS:** The sample was ionized by ESI. The monoisotopic mass was calculated from pseudo molecular ion [M+H]+ and is in agreement with the theoretically expected mass with a deviation of less than 1 ppm, confirming an elemental composition of C₂₀H₁₈N₃O₈Br.
M_{monoisotopic, experimental}: **507.0276** Da; M_{monoisotopic, theoretical}: **507.0277** Da

### b) Synthesis of 7

In an oven dried round bottom flask, **5** (7-diethylaminocoumarin-3-carboxylic acid, 1.0 equiv) and HATU (0.9 equiv) were transferred. Minimum amount of dry DMF was added and the mixture was stirred with a Teflon coated stirring bar under argon atmosphere at room temperature for 10-15 min to affect total dissolution. DIPEA (2.0 equiv) was then added to the stirred solution for a thorough mixing. Thereafter, mono boc-protected hydrazine (1.2 equiv) was added to the reaction mixture under argon atmosphere for 30 min. Coupled product was formed rapidly and reaction was quenched adding 50 µL TFA and purified via reverse phase HPLC. To access the installed hydrazine motif, the tert-butyl group required to be removed. It was thereafter treated with 50% TFA/DCM to furnish compound **6.** The HPLC purified 6 was then utilized for further coupling reaction. A complete characterization has been performed and data is as follows:

### Compound 6

**¹³C-NMR (125 MHz, d₆-DMSO):** δ **=** 161.60, 161.25, 157.01, 152.33, 147.16, 131.42, 110.08, 108.90, 107.57, 95.85, 44.28, 12.26
**¹H-NMR (600 MHz, d₆-DMSO):** δ = 9.44 (s, 1 H), 8.64 (s, 1 H), 7.68 (d, 1 H), 6.80 (dd, 1 H), 6.60 (d, 1 H), 4.60 (s, 2H), 3.48 q, 4H), 1.14 (t, 6H) **MS** (ESI pos.) calculated for C₁₄H₁₇N₃O₃: 275.0, Found 276.1 [M+H]⁺
Fmoc-Asp(O*^{t}*Bu)-OH (2.0 equiv) and HATU (1.9 equiv) were taken in a dry round bottom flask. Then minimum volume of dry DMF was added stirred for 15 min. DIPEA (4.0 equiv) was added and mixed thoroughly. Compound **6** (1.0 equiv) was added to the mixture and stirred for 1 h until the completion of the reaction. The reaction was then quenched with 15 µL of TFA and pure product was obtained after HPLC purification. The purified product was then subjected to 20% piperidine in DMF for the removal of fmoc-group. After fmoc removal, compound **7** was characterized analytically before the final step coupling reaction.

### Compound 7

**¹³C-NMR (125 MHz, d₆-DMSO):** δ = 168.16, 164.61, 161.46, 159.68, 157.47, 152.95, 148.44, 131.92, 110.46, 107.69, 107.18, 95.92, 81.61, 47.56, 44.41, 36.26, 27.66, 12.30
**¹H-NMR (600 MHz, d₆-DMSO):** δ = 11.28 (s, 1 H), 10.66 (s, 1 H), 8.72 (s, 1 H), 8.36 (s, 3H), 7.73 (d, 1 H), 6.85 (dd, 1 H), 6.65 (d, 1 H), 4.21 (m, 1 H), 3.50 (q, 4H), 2.90 (dd, 1 H), 2.77 (dd, 1 H), 1.44 (s, 9H), 1.15 (t, 6H) **MS** (ESI pos.) calculated for C₂₂H₃₀N₄O₆: 446.0, Found 447.1 [M+H]⁺

### c) Synthesis of 8

To a stirred solution of **4** (1.2 equiv) and HATU (1.1 equiv) in DMF, DIPEA (2.0 equiv) was added and mixed thoroughly. Compound **7** (1.0 equiv) was taken into 1 mL (500 µL x 2) DMF and added to the mixture at room temperature and stirred for 2 h. After complete consumption of **7,** the new coupled product was formed and was isolated by HPLC. The HPLC pure coupled product of **4** and **7** was then treated with 50% TFA/DCM for the removal of *tert*-butyl group on Asp to get the desired product **8** (**8a** or **8b** or **8c**). The final compound was further purified via preparative HPLC. A complete analytical characterization was performed for **8** and the analysis report is attached at the end of this supporting information. The analysis confirmed the structure of **8**.

### Probe III (8a):

**¹³C-NMR (125 MHz, d₆-DMSO):** δ = 171.48, 171.18, 169.44, 169.19, 167.99, 164.66, 161.37, 160.91, 160.60, 159.82, 157.38, 156.34, 152.79, 148.43, 148.23, 131.80, 131.75, 113.92, 113.77, 112.10, 110.34, 107.73, 107.65, 100.31, 95.94, 56.30, 52.10, 48.84, 48.16, 44.38, 40.63, 36.01, 30.36, 29.75, 25.34, 19.03, 12.30
**¹H-NMR (600 MHz, d₆-DMSO):** δ = 10.72 (d, 1 H), 10.43 (d, 1 H), 9.37 (d, 1 H), 8.89 (s, 1 H), 8.69 (s, 1 H), 8.54 (d, 1 H), 7.94 (d, 1 H), 7.72 (d, 1 H), 7.14 (d, 1 H), 7.06 (dd, 1 H), 6.84 (dd, 1 H), 6.65 (d, 1 H), 5.18 (dd, 1 H), 4.86 (m, 1 H), 4.72 (m, 1 H), 4.34 (m, 1 H), 3.91 (s, 3H), 3.50 (q, 4H), 3.35 (m, 1 H), 2.98 m, 1 H), 2.76 (dd, 1 H), 2.62 (m, 1 H), 2.60 (dd, 1 H), 2.15 (m, 1 H), 2.06 (m, 1 H), 1.89 (m, 1 H, 1.82 (m, 1 H), 1.68 (m, 1 H), 1.56 (m, 1 H), 1.15 (t, 6H)

HRMS: The sample was ionized by ESI. The monoisotopic mass was calculated from pseudo molecular ion [M+H]+ and is in agreement with the theoretically expected mass with a deviation of less than 1 ppm, confirming an elemental composition of C₃₉H₄₁N₇O₁₃.
M_{monoisotopic, experimental}: **815.27597** Da; _{Mmonoisotopic, theoretical}: **815.27623** Da

### Probe IV (8b):

**¹H-NMR (600 MHz, d₆-DMSO):** δ = 12.40 (s, 1H), 11.12 (s, 1H), 10.72 (s, 1 H), 10.42 (s, 1 H), 9.35 (d, 1 H), 8.83 (s, 1 H), 8.69 (s, 1 H), 8.53 (d, 1 H), 7.84 (d, 1 H), 7.72 (d, 1 H), 6.89 (dd, 1 H), 6.84 (dd, 1 H), 6.81 (d, 1H), 6.64 (d, 1 H), 5.18 (dd, 1 H), 4.85 (m, 1 H), 4.72 (m, 1 H), 4.33 (m, 1 H), 3.50 (q, 4H), 3.34 (m, 1 H), 2.97 (m, 1 H), 2.75 (dd, 1 H), 2.61 (m, 1 H), 2.60 (dd, 1 H), 2.14 (m, 1 H), 2.05 (m, 1 H), 1.88 (m, 1 H), 1.81 (m, 1 H), 1.68 (m, 1 H), 1.56 (m, 1 H), 1.15 (t, 6H)
**HRMS:** The sample was ionized by ESI. The monoisotopic mass was calculated from pseudo molecular ion [M+H]+ and is in agreement with the theoretically expected mass with a deviation of less than 1 ppm, confirming an elemental composition of C₃₈H₃₉N₇O₁₃.
M_{monoisotopic}, ₑₓₚₑᵣᵢₘₑₙₜₐₗ: **801.2612** Da; _{Mmonoisotopic, theoretical}: **801.2606** Da

### Probe V (8c):

**¹H-NMR (600 MHz, d₆-DMSO):** δ = 12.4 (s, 1 H), 12.0 (s, 1 H), 10.72 (d, 1 H), 10.43 (d, 1 H), 9.32 (d, 1 H), 8.83 (s, 1 H), 8.69 (s, 1 H), 8.53 (d, 1 H), 8.26 (s, 1 H), 7.72 (d, 1 H), 6.95 (s, 1 H), 6.84 (dd, 1 H), 6.65 (d, 1 H), 5.17 (dd, 1 H), 4.85 (m, 1 H), 4.72 (m, 1 H), 4.33 (m, 1 H), 3.50 (q, 4H), 3.33 (m, 1H), 2.97 (m, 1 H), 2.75 (dd, 1 H), 2.61 (m, 1 H), 2.59 (dd, 1 H), 2.14 (m, 1H), 2.05 (m, 1 H), 1.88 (m, 1 H), 1.81 (m, 1 H), 1.67 (m, 1 H), 1.56 (m, 1H), 1.15 (t, 6H)

HRMS: The sample was ionized by ESI. The monoisotopic mass was calculated from pseudo molecular ion [M+H]+ and is in agreement with the theoretically expected mass with a deviation of less than 2 ppm, confirming an elemental composition Of C₃₈H₃₈N₇O₁₃Br.
M_{monoisotopic, experimental}: **879.16984** Da; _{Mmonoisotopic, theoretical}: **879.17109** Da

### 8. Scheme for the synthesis of Probe VI & Probe VII

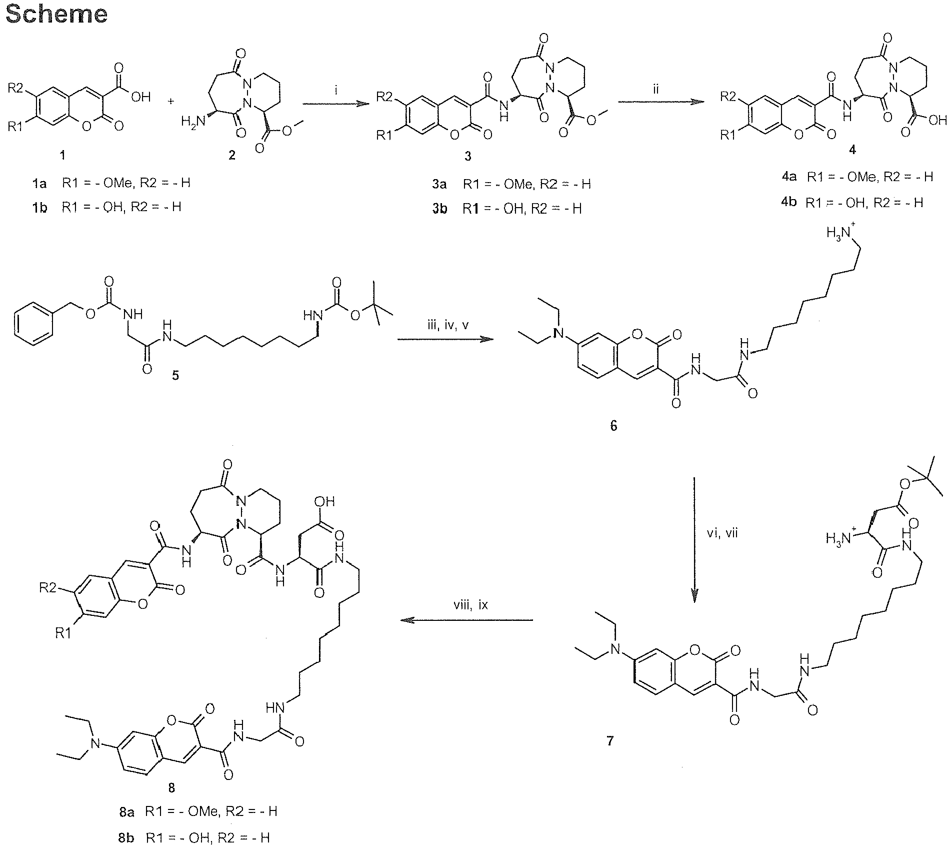

**Reagents and conditions:** (i) HATU, DIPEA, DMF, 1 h, RT, (ii) LiOH / THF:Water:Methanol (2:1:1) RT, 4 h, (iii) H₂, Pd/C, EtOH, (iv) 7-diethylaminocoumarin-3-carboxylic acid, HATU, DIPEA, DMF, 1 h, RT, (v) 50% TFA/DCM, 15 min, (vi) Fmoc-Asp(O^{t}Bu)-OH, HATU, DIPEA, DMF, RT, 1 h, (vii) 20% piperidine in DMF, 1 h (viii) **4**, HATU, DIPEA, DMF, RT, 2h, (ix) 50% TFA/DCM, 15 min.

### 9. Synthesis of 8

### a) Synthesis of 4a, and 4b was carried analogously as the synthesis of compound 4 of probe I using starting material 1 a and 1 b.

### b) Synthesis of 7

In an oven dried round bottom flask, **5** (Commercially available amino acid building block, 1.0 equiv) and 5% Pd/C (0.1 equiv) were transferred. Minimum amount of dry EtOH was added and the mixture was stirred with a Teflon coated stirring bar at room temperature. The mixture was then placed under hydrogen (H₂). A thorough alternative vacuam and hydrogen cycles (5 cycles each) were deployed for ensuring complete oxygen-free environment. Hydrogenation of **5** for removal of Cbz group was achieved using 10 mbar pressure of H₂ for 60 min. using 5% Pd/C as catalyst. After completion of reaction (checked by LC-MS), the mixture was filtered through a special filter (Spritzenfilter PET (Carl Roth GmbH and Co), 0.45 µm, 25 mm) to get rid of carbon particles. The mixture was evaporated under vacuam and used for the next coupling step without further purification. Thereafter, 7-diethylaminocoumarin-3-carboxylic acid (1.2 equiv), HATU (1.1 equiv), and DIPEA (2.0 equiv) were added using minimum volume of dry DMF and the mixture was stirred for 1 h for the completion of the reaction. The coupled product was then purified via preparative HPLC. The pure product was thereafter treated with 50% TFA/DCM to furnish compound **6**. The HPLC purified **6** was then utilized for further coupling reaction. A complete characterization has been performed and data is as follows:

### Compound 6

**¹³C-NMR (125 MHz, d₆-DMSO):** δ = 168.03, 162.12, 161.54, 157.25, 152.46, 147.68, 131.58, 110.11, 109.15, 107.57, 95.84, 44.30, 42.50, 38.80, 38.47, 28.99, 28.41, 28.36, 26.91, 26.16, 25.66, 12.27
**¹H-NMR (600 MHz, d₆-DMSO):** δ = 8.99 (t, 1 H), 8.65 (s, 1 H), 7.98 (t, 1 H), 7.68 (d, 1 H), 7.67 (broad, 3H), 6.81 (dd, 1 H), 6.62 (d, 1 H), 3.93 (d, 2H), 3.49 (q, 4H), 3.07 (dt, 2H), 2.77 (m, 2H), 1.51 (m, 2H), 1.41 (m, 2H), 1.30-1.24 (m, 8H), 1.14 (t, 6H)
**MS** (ESI pos.) calculated for C₂₄H₃₇N₄O₄: 445.0, Found 446.2 [M+H]⁺

Fmoc-Asp(O*^{t}*Bu)-OH (2.0 equiv) and HATU (1.9 equiv) were taken in a dry round bottom flask. Then minimum volume of dry DMF was added stirred for 15 min. DIPEA (4.0 equiv) was added and mixed thoroughly. Compound **6** (1.0 equiv) was added to the mixture and stirred for 1 h until the completion of the reaction. The reaction was then quenched with 15 µL of TFA and pure product was obtained after HPLC purification. The purified product was then subjected to 20% piperidine in DMF for the removal of fmoc-group. After fmoc removal, compound **7** was re-purified using HPLC. Pure **7** was then characterized analytically before the final step coupling reaction.

### Compound 7

**¹³C-NMR (125 MHz, d₆-DMSO):** δ = 168.36, 168.05, 167.00, 162.13, 161.54, 157.26, 152.46, 147.67, 131.58, 110.10, 109.17, 107.58, 95.84, 81.37, 48.83, 44.31, 42.51, 38.85, 38.52, 36.19, 29.03, 28.68, 28.60, 27.61, 26.30, 26.18, 12.26
**¹H-NMR (600 MHz, d₆-DMSO):** δ = 8.98 (t, 1 H), 8.65 (s, 1 H), 8.37 (t, 1 H), 8.20 (broad, 3H), 7.99 (t, 1 H), 7.67 (d, 1 H), 6.80 (dd, 1 H), 6.61 (d, 1 H), 3.97 (m, 1 H), 3.93 (d, 2H), 3.48 (q, 4H), 3.14 (m, 1 H), 3.06 (dt, 2H), 3.04 (m, 1 H), 2.77 (dd, 1 H), 2.73 (dd, 1 H), 1.41 (s, 9H), 1.40 (m, 2H), 1.39 (m, 2H), 1.25 (m, 8H), 1.14 (t, 6H)
**MS** (ESI pos.) calculated for C₃₂H₅₀N₅O₇: 616.0, Found 617.2 [M+H]⁺

### c) Synthesis of 8

To a stirred solution of **4** (1.2 equiv) and HATU (1.1 equiv) in DMF, DIPEA (2.0 equiv) was added and mixed thoroughly. Compound **7** (1.0 equiv) was taken into 1 mL (500 µL x 2) DMF and added to the mixture at room temperature and stirred for 2 h. After complete consumption of **7,** the new coupled product was formed and was isolated by HPLC. The HPLC pure coupled product of **4** and **7** was then treated with 50% TFA/DCM for the removal of *tert*-butyl group on Asp to get the desired product **8 (8a or 8b).** The final compound was further purified via preparative HPLC. A complete analytical characterization was performed for 8 and the analysis report is attached at the end of this supporting information. The analysis confirmed the structure of **8**.

### Probe VII

**¹³C-NM (125 MHz, d₆-DMSO):** δ = 171.53, 171.48, 169.85, 169.45, 169.06, 168.04, 163.90, 162.14, 161.55, 161.02, 160.75, 157.26, 156.42, 152.44, 148.57, 147.69, 132.20, 131.59, 114.41, 112.74, 111.07, 110.09, 109.18, 107.59, 101.81, 95.86, 52.18, 49.64, 48.78, 44.31, 42.52, 40.63, 38.55, 36.10, 30.43, 29.73, 29.05, 28.93, 28.69, 26.35, 26.19, 25.27, 18.98, 12.29
**¹H-NMR (600 MHz, d₆-DMSO):** δ = 12.3 (s, 1 H), 11.12 (s, 1 H), 9.34 (d, 1H), 8.98 (t, 1H), 8.82 (s, 1H), 8.65 (s, 1H), 8.42 (d, 1H), 7.97 (t, 1H), 7.83 (d, 1 H), 7.82 (t, 1 H), 7.68 (d, 1 H), 6.88 (dd, 1 H), 6.81 (d, 1 H), 6.80 (dd, 1 H), 6.62 (d, 1 H), 5.15 (dd, 1 H), 4.84 (m, 1 H), 4.50 (m, 1 H), 4.32 (m, 1 H), 3.92 (d, 2H), 3.48 (q, 4H), 3.32 (m, 1 H), 3.07 (dt, 2H), 3.02 (dt, 2H), 2.98 (m, 1 H), 2.65 (dd, 1H), 2.61 (m, 1 H), 2.50 (dd, 1 H), 2.12 (m, 1 H), 1.99 (m, 1 H), 1.87 (m, 1 H), 1.79 (m, 1 H), 1.64 (m, 1 H), 1.55 (m, 1 H), 1.39 (m, 2H), 1.36 (m, 2H), 1.26-1.20 (m, 8H), 1.14 (t, 6H)
**HRMS:** The sample was ionized by ESI. The monoisotopic mass was calculated from pseudo molecular ion [M+H]+ and is in agreement with the theoretically expected mass with a deviation of less than 1 ppm, confirming an elemental composition of C₅₆H₇₃N₇O₁₅.
M_{monoisotopic, experimental}: **970.40663** Da; _{Mmonoisotopic, theoretical}: **970.40724** Da

### Probe VI

**¹³C-NMR (125 MHz, d₆-DMSO)**: δ = 171.53, 171.50, 169.89, 169.43, 169.08, 168.06, 164.67, 162.15, 161.56, 160.91, 160.60, , 157.27, 156.34, 152.45, 148.42, 147.70, 131.76, 131.61, 113.90, 113.78, 112.09, 110.11, 109.20, 107.60, 100.31, 95.87, 56.31, 52.23, 49.70, 48.83, 44.33, 42.53, 40.65, 38.57 (2C), 36.13, 30.39, 29.74, 29.06, 28.94, 28.70 (2C), 26.37, 26.20, 25.31, 19.00, 12.30
**¹H-NMR (600 MHz, d₆-DMSO)**: δ = 9.35 (d, 1 H), 8.98 (t, 1 H), 8.87 (s, 1 H), 8.65 (s, 1 H), 8.45 (d, 1 H), 8.00 (t, 1 H), 7.93 (d, 1 H), 7.84 (t, 1 H), 7.68 (d, 1 H), 7.13 (d, 1 H), 7.05 (dd, 1 H), 6.80 (dd, 1 H), 6.61 (d, 1 H), 5.15 (dd, 1 H), 4.85 (m, 1 H), 4.50 (m, 1 H), 4.32 (m, 1 H), 3.92 (d, 2H), 3.90 (s, 3H), 3.47 (q, 4 H), 3.33 (m, 1 H), 3.06 (dt, 2H), 3.01 (dt, 2H), 2.98 (m, 1 H), 2.65 (dd, 1 H), 2.61 (m, 1 H), 2.51 (dd, 1 H), 2.13 (m, 1 H), 2.00 (m, 1 H), 1.87 (m, 1 H), 1.80 (m, 1 H), 1.64 (m, 1 H), 1.55 (m, 1 H), 1.39 (m, 2H), 1.36 (m, 2H), 1.26-1.20 (m, 8H), 1.13 (t, 6H)
**HRMS:** The sample was ionized by ESI. The monoisotopic mass was calculated from pseudo molecular ion [M+H]+ and is in agreement with the theoretically expected mass with a deviation of less than 2 ppm, confirming an elemental composition of C₄₉H₆₀N₈O₁₄.
M_{monoisotopic}, ₑₓₚₑᵣᵢₘₑₙₜₐₗ: **984.42438** Da; _{Mmonoisotopic, theoretical}: **984.42289** _{Da}

### 5. Biological Data Summary

### Cellular assay

THP-1/U937 cells were differentiated into macrophages with PMA (30 ng/ml) for 48 hours in a 96-well plate with a transparent bottom. As a control, the caspase inhibitor zVAD was added 2 hours before adding other reagents. Cells were stimulated with 5 mM ATP or 100 ng/µl LPS or a combination of both. As negative control nonstimulated cells were used. The probe was then added to the wells at 5 µM final concentration. Fluorescence was then measured at appropriate excitation/emission wavelengths for 2 hours. All experiments were performed in 6 replicates, average (dRFU/dt) was calculated for each series. The probe successfully detected caspase-1 activity in cells.

### Kinetic characterization

Kₘ and k_{cat}/Kₘ values were determined using standard methods (Kindermann et al., 2010; Rozman Pungercar et al., 2003). Caspase-1 was added to increasing concentrations of the probe and initial velocities of probe hydrolysis were calculated from the slopes of the lines of the fluorescence increase. Kₘ and k_{cat} (Vmax) values were then determined by nonlinear regression analysis using the Prism program.

### Cell permeability assay

THP-1 cells were differentiated with PMA in 4-well glass chambers for 48 hours. 2 hours before the assay zVAD-fmk was added to negative controls. Probes were then added to the cells in 5 µM final concentration and the probe uptake was studied by fluorescence microscopy. The probes can be seen in the cytoplasm and occasionally in the vesicle-like structures suggesting that the probe intake is a combination of passive entry and endocytosis (bright spots are the vesicles marked with a dark arrow). The probes stayed in the cytoplasm (nucleus of the cell is marked with a bright arrow).

**Results of biological assays**

| | |
|---|---|
| Probe III: | - Concentration dependent FRET change |
| | - Cell permeability |
| | - No sign of toxicity |
| | - Efficient Kcat/Km |
| | - Superiorly specific |
| | |
| Probe II: | - Rapid staining of cell membrane |
| | |
| Probe I: | - Cellular entry within 5-7 min. |

**Results of kinetic characterization of probes:**

| Probe | K_{M} | k_{cat}/K_{M} |
|---|---|---|
| Probe III | **4.2 µM** | **8600** |
| Prob VI | **2.3 µM** | **27500** |
| Probe V | **5.9 µM** | **35300** |
| Probe IV | **6.5 µM** | **7800** |
| Probe VII | **16.2 µM** | **10000** |

## Claims

1. Molecular probe for a cysteine protease of the formula (I)
L1-Lₙ-A-CO-NR₁-R₂W-L2 (I)
wherein
A is a group recognizable by caspase-1;
R₁ is hydrogen, hydroxy, C₁-C₁₈-alkyl, preferably C₁-C₆-alkyl, more preferably C₁-, C₂-, C₃-alkyl, wherein optionally one or more carbon atoms are substituted by halogen; C₃-C₁₈-alkyl, wherein one or more carbon atoms are replaced by an oxygen atom; alkylaryl such as C₁-, C₂-, C₃-aryl or -NHCOR₄;
R₂ is a bond or NR₃ wherein R₃ is hydrogen, hydroxy, C₁-C₁₈-alkyl, preferably C₁-C₆-alkyl, more preferably C₁-, C₂-, C₃-alkyl, wherein optionally one or more carbon atoms are substituted by halogen; C₃-C₁₈-alkyl, wherein one or more carbon atoms are replaced by an oxygen atom; alkylaryl, such as C₁-, C₂-, C₃-aryl; or -NHCOR₄ or wherein R₁ and R₃ form together with the nitrogen atoms to which they are attached to a 3-, 4-, 5-, 6-membered saturated or unsaturated ring, optionally with one or more, preferably two, additional hetereoatoms selected from oxygen or nitrogen;
R₄ is alkyl, aryl, or alkylaryl;
W is a straight or branched alkylene group with 1 to 300 carbon atoms, wherein optionally
a) one or more carbon atoms are replaced by a nitrogen atom carrying a hydrogen atom, and the adjacent carbon atoms are substituted by oxo, representing an amide function -NH-CO-; and/or
b) one or more carbon atoms are substituted by oxo, representing a carbonyl function -CO;
c) the bond between two adjacent carbon atoms is a double or triple bond; and/or
d) one or more carbon atoms are replaced by an ester function -O-CO-; L is a group selected from 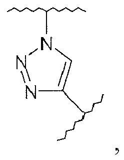 -(NRx)-, -O-, -C=N-, -C(=O)-, -C(=O)-NH-, -NH-C(=O)-, -CRx=CRy-, -C=C- and phenyl, wherein Rx and Ry are independently H or (C₁-C₆)alkyl, preferably L is -C(=O)-;
L1 and L2 are, independent of each other at least one label; and n is 0 or 1.

2. Molecular probe according to claim 1 wherein
wherein
R₁ is hydrogen;
R₂ is a bond; and
W is a straight or branched alkylene group with 10-20 carbon atoms, wherein one or more carbon atoms are replaced by a nitrogen atom carrying a hydrogen atom, and the adjacent carbon atoms are substituted by oxo, representing an amide function -NH-CO-.

3. Molecular probe according to claims 1 or 2 wherein W is a straight alkylene group with 13 carbon atoms, particularly wherein one or more carbon atoms are replaced as follows -(CH₂)₈-NH-CO-CH₂-NH-CO-.

4. Molecular probe according to claim 1 with the formula (III)
L1-Lₙ-A-CO-NR₁-NR₃-W-L2 (III)
wherein
R₁ and R₃ form together with the nitrogen atoms to which they are attached to a 3-, 4-, 5-, 6-membered saturated or unsaturated ring, optionally with one or more, preferably two, additional hetereoatoms selected from oxygen or nitrogen, or
R₁ and R₃ are independent from each other hydrogen, hydroxy, C₁-C₁₈-alkyl, preferably C₁-C₆-alkyl, more preferably C₁-, C₂-, C₃-alkyl, wherein optionally one or more carbon atoms are substituted by halogen; C₃-C₁₈-alkyl, wherein one or more carbon atoms are replaced by an oxygen atom; alkylaryl such as C₁-, C₂-, C₃-aryl; or -NHCOR₄.

5. Molecular probe according to claim 4 wherein:
a) R₁ and R₃ are each hydrogen;
b) R₁ is hydrogen and R₃ is selected from alkyl, alkylaryl and NHCOR₄;
c) R₁ is selected from alkyl, alkylaryl and -NHCOR₄ and R₃ is hydrogen; or
d) R₁ and R₃ are each alkyl, alkylaryl or -NHCOR₄.

6. Molecular probe according to claims 4 or 5 wherein:
(a) W is a straight or branched alkylene group with 1 to 15 carbon atoms, wherein one or more carbon atoms are substituted by an oxo function, representing a carbonyl function -C(O), particularly wherein W is a straight alkylene group with 1 carbon atom wherein said carbon atom is substituted by an oxo function, representing a carbonyl function -C(O); or
(b) W is a straight or branched alkylene group with 20-35 carbon atoms, wherein one or more carbon atoms are replaced by a nitrogen atom carrying a hydrogen atom, and the adjacent carbon atoms are substituted by oxo, representing an amide function -NH-CO- and one or more carbon atoms are substituted by oxo, representing a carbonyl function -C(O), particularly wherein W is -C(O)CH(Z)NH-C(O)- wherein Z is (CH₂)_{q}-NHC(O)(CH₂)ₘCH₃, wherein q is preferably 4 and m is preferably 14; or
(c) W is a straight or branched alkylene group with 5-10 carbon atoms, wherein one or more carbon atoms are substituted by oxo, representing a carbonyl function -C(O) and one or more carbon atoms are replaced by an ester function -O-CO-, particularly wherein W is - C(O)(CH₂)ₚC(O)OCH₂- and p is preferably 3.

7. Molecular probe according to any one of claims 1 to 6 wherein L1 is selected from 7-methoxycoumarin, 6-bromo-,7-hydroxycoumarin, and 7-hydroxycoumarin, and/or L2 is selected from 7-N,N-dialkylcoumarin, preferably 7-N,N-diethylcoumarin or 7-N,N-dioctanylcoumarin.

8. Molecular probe according to any one of claims 1 to 7 wherein A is selected from the group consisting of:
| | |
|---|---|
| 1. | 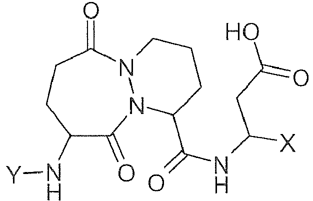 |
| | preferably in the configuration: |
| | 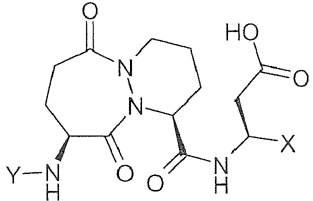 |
| 2. | 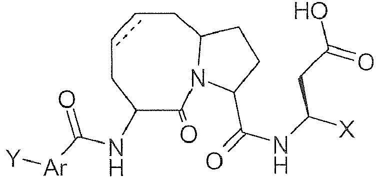 |
| | wherein Ar is an aryl or heteroaryl group selected from phenyl, benzothiophene, isoquinolyl, cinnamyl, naphthyl, which is optionally once or independently twice substituted by methoxy, chloro, methyl, CF₃, and wherein |
| | 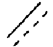 |
| | means either a single or a double bond. |
| 3. | 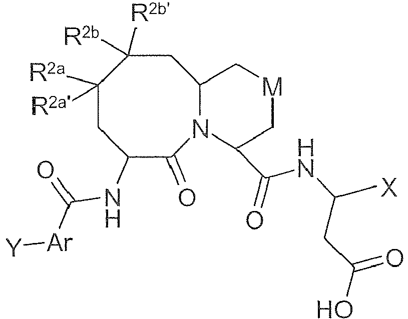 |
| | preferably in the all-(S) configuration, |
| | wherein Ar is aryl or heteroaryl; |
| | M is CH₂, O or NR₉ wherein R₉ is hydrogen or (C₁-C₆)alkyl, aryl, heteroaryl, heterocyclyl; |
| | R^{2a}, R^{2a'}, R^{2b} and R^{2b'} are each independently hydrogen, hydroxyl, N(R⁶)₂, halogen, (C₁-C₄)alkyl, (C₁-C₄)alkoxy, and mixtures thereof wherein R⁶ is hydrogen, (C₁-C₆)alkyl, cycloalkyl, (C₆-C₁₀)aryl; or R^{2a} and R^{2b} can taken together to form a double bond. |
| 4. | 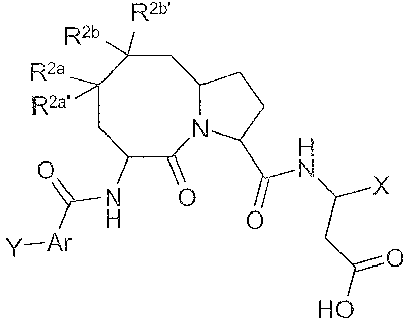 |
| | preferably in the all-(S) configuration, |
| | wherein Ar is aryl or heteroaryl; |
| | R^{2a}, R^{2a'}, R^{2b} and R^{2b'} are each independently hydrogen, hydroxyl, N(R⁶)₂, halogen, (C₁-C₄)alkyl, (C₁-C₄)alkoxy, and mixtures thereof wherein R⁶ is hydrogen, (C₁-C₆)alkyl, cycloalkyl, (C₆-C₁₀)aryl; or R^{2a} and R^{2b} can taken together to form a double bond. |
wherein in each of the above compounds X is -CO-NR₁-R₂-W-L2; Y is L1-Lₙ-; and R₁, R₂, Lₙ, W, L1 and L2 are defined as in any one of the preceding claims.

9. Molecular probe according claim 8 wherein A is 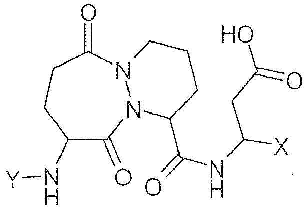 particularly in the configuration of 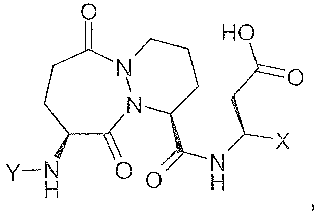 wherein in each of the above compounds X is -CO-NR₁-R₂-W-L2; Y is L1-Lₙ-; and R₁, R₂, Lₙ, W, L1 and L2 are defined as in any one of the preceding claims.

10. Molecular probe according to claim 9 selected from

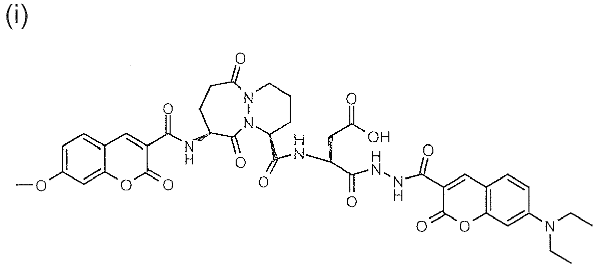

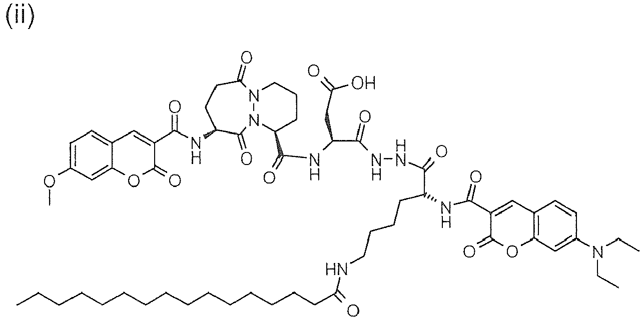

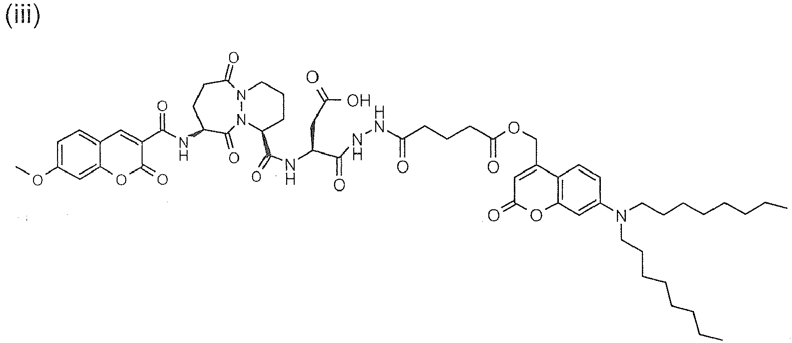

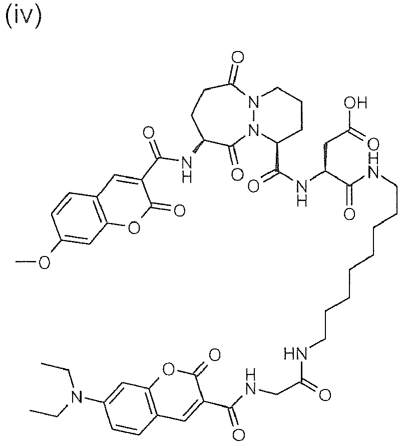

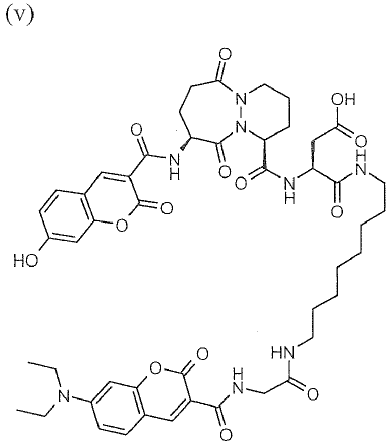

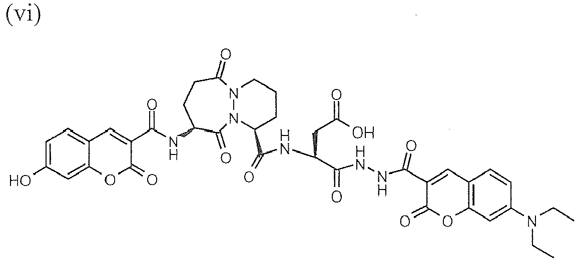

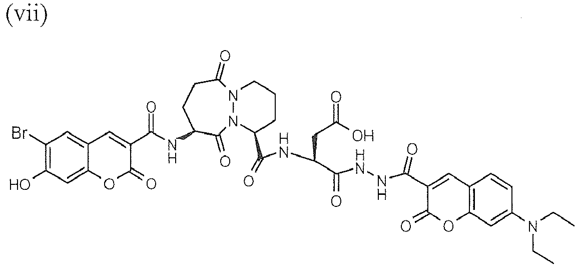

11. Molecular probe according to any one of claims 1 to 10 for use in medicine.

12. Molecular probe according to any one of claims 1 to 10 for use in diagnosis, preferably for imaging a living organism and/or for the in vivo-identification of biomarkers, particularly biomarkers for inflammatory diseases.

13. Molecular probe according to claim 12 wherein the use is for imaging a living organism and comprises:
(a) administering to said organism a probe of the formula (I),
(b) exposing said organism to electromagnetic radiation which excites a fluorophore or non-quenched fluorophore to produce a detectible signal, and
(c) detecting said signal and creating an image thereby.

14. Use of a probe according to any one of claims 1-10 for the identification of biomarkers, preferably biomarkers for inflammatory diseases.

15. Use of a probe according to any one of claims 1-10 for molecular imaging in vitro, in cell-culture experiments, or ex-vivo experiments.
